(19) Europäisches Patentamt
European Patent Office
Office européen des brevets

(11) **EP 4 697 353 A2**

(12) **EUROPEAN PATENT APPLICATION**

(43) Date of publication:
**18.02.2026 Bulletin 2026/08**

(21) Application number: 25209720.9

(22) Date of filing: **29.01.2021**

(51) International Patent Classification (IPC):
***G16H 40/63*** *(2018.01)*

(52) Cooperative Patent Classification (CPC):
**A61M 16/024; A61B 5/02055; A61B 5/0816;**
**A61B 5/087; G16H 20/40; G16H 40/63;**
**G16H 40/67; G16H 50/20;** A47G 9/10; A61B 5/097;
A61B 5/4809; A61B 5/4815; A61B 5/4818;
A61M 16/0066; A61M 16/06;     (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **31.01.2020 US 202062968889 P**
**30.09.2020 US 202063198137 P**

(62) Document number(s) of the earlier application(s) in
accordance with Art. 76 EPC:
**21703315.8 / 4 096 757**

(71) Applicant: **ResMed Sensor Technologies Limited**
**Dublin D18 T6T7 (IE)**

(72) Inventors:
• **SHOULDICE, Redmond**
**Dublin, D18 T6T7 (IE)**
• **MCMAHON, Stephen**
**Dublin, D18 T6T7 (IE)**

• **LYON, Graeme**
**Dublin, D18 T6T7 (IE)**
• **TIRON, Roxana**
**Dublin, D18 T6T7 (IE)**
• **DENIA, Marta Perez**
**Dublin, D18 T6T7 (IE)**
• **COFFEY, Sam**
**Dublin, D18 T6T7 (IE)**

(74) Representative: **Mathys & Squire**
**Theatinerstraße 8**
**80333 München (DE)**

Remarks:
•This application was filed on 20.10.2025 as a
divisional application to the application mentioned
under INID code 62.
•Claims filed after the date of filing of the application/
the date of receipt of the divisional application (Rule
68(4) EPC).

(54)     **SYSTEM FOR DETECTING MOUTH LEAK**

(57)     The present disclosure relates to a method for determining a mouth leak status associated with a user of a respiratory device is disclosed. Airflow data associated with the user of the respiratory device is received. The respiratory device is configured to supply pressurized air to an airway of the user during a therapy session. The airflow data includes pressure data. The airflow data associated with the user is analyzed. Based at least in part on the analysis, the mouth leak status associated with the user is determined. The mouth leak status is indicative of whether or not air is leaking from a mouth of the user.

FIG. 1

EP 4 697 353 A2

(52) Cooperative Patent Classification (CPC): (Cont.)
A61M 16/109; A61M 16/1095; A61M 16/14;
A61M 16/16; A61M 16/161; A61M 2016/0027;
A61M 2016/0036; A61M 2016/1025;
A61M 2021/0083; A61M 2205/15; A61M 2205/18;
A61M 2205/215; A61M 2205/3303;
A61M 2205/3306; A61M 2205/332;
A61M 2205/3331; A61M 2205/3368;
A61M 2205/3375; A61M 2205/3553;
A61M 2205/3569; A61M 2205/3584;
A61M 2205/3592; A61M 2205/505; A61M 2205/52;
A61M 2205/581; A61M 2205/582; A61M 2205/583;
A61M 2209/088; A61M 2210/0625; A61M 2230/04;
A61M 2230/10; A61M 2230/201; A61M 2230/205;
A61M 2230/208; A61M 2230/30; A61M 2230/40;
A61M 2230/42; A61M 2230/43; A61M 2230/50;
A61M 2230/60; A61M 2230/63; A61M 2230/65

**Description**

**CROSS-REFERENCE TO RELATED APPLICATIONS**

This application claims the benefit of, and priority to, U.S. Provisional Patent Application No. 62/968,889 filed on January 31, 2020, and U.S. Provisional Patent Application No. 63/198,137 filed on September 30, 2020, each which is hereby incorporated by reference herein in its entirety.

**TECHNICAL FIELD**

The present disclosure relates generally to systems and methods for determining a mouth leak status for a user, and more particularly, to systems and methods for determining a mouth leak status for the user based on acoustic and/or airflow data generated during a sleep session of the user.

**BACKGROUND**

Breathing not only provides oxygen to our bodies, but also releases carbon dioxide and waste. The nose and mouth form two air passageways to our lungs, and can facilitate gas exchange. People may breathe through their mouth at night if their nasal air passageway is obstructed (either completely blocked or partially blocked). Some people develop a habit of breathing through their mouth instead of their nose even after the nasal obstruction clears. For some people with sleep apnea, it may become a habit to sleep with their mouth open to accommodate their need for oxygen.
Furthermore, when sleep apnea patients begin CPAP therapy using a nasal mask or nasal pillows, they may inadvertently breathe through their mouth ("mouth leak"). For example, when the delta between the pressure in the mouth and the atmospheric pressure exceeds a threshold, the mouth (e.g., the lips) may pop open to normalize the pressure. The lips may close again on inhalation. This may not wake the patients, but can lead to dry mouth, dry lips, and discomfort when they wake. Some patients will not tolerate this for long, and are highly likely to stop their much needed therapy. Therefore, it is desirable to detect and/or monitor patients that experience mouth leak during respiratory therapy.
The present disclosure is directed to solving these and other problems.

**SUMMARY**

According to some implementations of the present disclosure, a system includes a memory storing machine-readable instructions and a control system including one or more processors. The control system is configured to execute the machine-readable instructions to: receive, from a microphone, first acoustic data associated with a user of a respiratory device; analyze the first acoustic data associated with the user; and determine a mouth leak status based, at least in part, on the analysis of the first acoustic data. The respiratory device is configured to supply pressurized air to an airway of the user during a sleep session. The mouth leak status is indicative of air leaking from a mouth of the user.
According to some implementations of the present disclosure, a system includes a memory storing machine-readable instructions and a control system including one or more processors. The control system is configured to execute the machine-readable instructions to: receive, from a microphone, acoustic data associated with a user of a respiratory device; and process, using a machine learning algorithm, the acoustic data to output a mouth leak status for the user. The respiratory device being configured to supply pressurized air to an airway of the user during a sleep session. The mouth leak status is indicative of air leaking from a mouth of the user.
According to some implementations of the present disclosure, a system includes a memory storing machine-readable instructions and a control system including one or more processors. The control system is configured to execute the machine-readable instructions to: receive, from a microphone, acoustic data associated with a user of a respiratory device during a plurality of sleep sessions; receive pressure data associated with pressurized air supplied to an airway of the user during the plurality of sleep sessions; analyze the acoustic data to determine a mouth leak status of the user for each sleep session of the plurality of sleep sessions; and determine, based at least in part on (i) the mouth leak status of the user for each sleep session of the plurality of sleep sessions and (ii) the pressure data, an optimal inhalation pressure and an optimal exhalation pressure for the user. The microphone is associated with the user of the respiratory device. The respiratory device is configured to supply the pressurized air to the airway of the user. The acoustic data includes inhalation acoustic data and exhalation acoustic data. The pressure data includes inhalation pressure data and exhalation pressure data. The mouth leak status is indicative of air leaking from a mouth of the user.
According to some implementations of the present disclosure, a system includes a memory storing machine-readable instructions and a control system including one or more processors. The control system is configured to execute the machine-readable instructions to: receive, from a microphone, acoustic data associated with a user during a plurality of sleep sessions; receive, from a sensor, physiological data associated with the user for each sleep session of the plurality of sleep sessions; analyze the acoustic data to determine a mouth leak status of the user for each sleep session of the plurality of sleep sessions; and train a machine learning algorithm with (i) the mouth leak status of the user for each sleep session of the plurality of sleep sessions and (ii) the physiological data, such that the machine learning algorithm is configured to: receive as an input current physiological data associated with a current sleep session; and determine as an output an estimated mouth leak status for the current sleep session. The microphone is associated with the user of a respiratory device. The respiratory device is configured to supply pressurized air to an airway of the user. The mouth leak status is indicative of air leaking from a mouth of the user.

According to some implementations of the present disclosure, a method for determining a mouth leak status associated with a user of a respiratory device is disclosed. Airflow data associated with the user of the respiratory device is received. The respiratory device is configured to supply pressurized air to an airway of the user during a therapy session. The airflow data includes pressure data. The airflow data associated with the user is analyzed. Based at least in part on the analysis, the mouth leak status associated with the user is determined. The mouth leak status is indicative of whether or not air is leaking from a mouth of the user.

According to some implementations of the present disclosure, a system includes a control system having one or more processors, and a memory having stored thereon machine readable instructions. The control system is coupled to the memory. Any of the methods disclosed above, and further described herein, is implemented when the machine executable instructions in the memory are executed by at least one of the one or more processors of the control system.

According to some implementations of the present disclosure, a system for determining a mouth leak status associated with a user of a respiratory device includes a control system having one or more processors configured to implement any of the methods disclosed above and further described herein.

According to some implementations of the present disclosure, a computer program product includes instructions which, when executed by a computer, cause the computer to carry out any of the methods disclosed above and further described herein. In some implementations, the computer program product is a non-transitory computer readable medium.

The above summary is not intended to represent each implementation or every aspect of the present disclosure. Additional features and benefits of the present disclosure are apparent from the detailed description and figures set forth below.

## BRIEF DESCRIPTION OF THE DRAWINGS

FIG. 1 is a functional block diagram of a system for determining a mouth leak status for a user, according to some implementations of the present disclosure;

FIG. 2A is a perspective view of at least a portion of the system of FIG. 1, a user wearing a full face mask, and a bed partner, according to some implementations of the present disclosure;

FIG. 2B is a perspective view of at least a portion of the system of FIG. 1, a user wearing a nasal mask, and a bed partner, according to some implementations of the present disclosure;

FIG. 3 is a process flow diagram for a method of determining a mouth leak status for a user, according to some implementations of the present disclosure;

FIG. 4A illustrates a visual indicator of a mouth leak rating for a user on a display device, according to some implementations of the present disclosure;

FIG. 4B illustrates a visual indicator of a message associated with a mouth leak status of a user on a display device, according to some implementations of the present disclosure;

FIG. 4C illustrates a user interface displayed on a display device for receiving user feedback from a user, according to some implementations of the present disclosure;

FIG. 5 is a process flow diagram for a method of determining an optimal inhalation pressure and an optimal exhalation pressure for a user, according to some implementations of the present disclosure;

FIG. 6 is a process flow diagram for a method of estimating a mouth leak status for a user using a machine learning algorithm, according to some implementations of the present disclosure;

FIG. 7 a process flow diagram for a method for determining a mouth leak status associated with a user of a respiratory device, according to some implementations of the present disclosure;

FIG. 8 illustrates a first breath while a user is breathing normally and a second breath while the user is exhaling through mouth, according to some implementations of the present disclosure;

FIG. 9 illustrates a plurality of features identified within a breath cycle, according to some implementations of the present disclosure;

FIG. 10A illustrates lab data measured during a therapy session of a user displaying valve-like mouth leak, mask leak, and continuous mouth leak, according to some implementations of the present disclosure;

FIG. 10B illustrates a portion of the lab data of FIG. 10 of the user displaying the valve-like mouth leak, according to some implementations of the present disclosure;

FIG. 10C illustrates a portion of the lab data of FIG. 10 of the user displaying the mask leak, according to some implementations of the present disclosure;

FIG. 10D illustrates a portion of the lab data of FIG. 10 of the user displaying the continuous mouth leak, according to some implementations of the present disclosure;

FIG. 11 illustrates a histogram of epochs with mouth leak in terms of unintentional leak levels, according to some implementations of the present disclosure;

FIG. 12A illustrates actual mouth leak duration, according to some implementations of the present disclosure;

FIG. 12B illustrates predicted mouth leak duration, according to some implementations of the present disclosure;

FIG. 13 illustrates proportions of scored mouth leak in terms of block duration, according to some implementations of the present disclosure;

FIG. 14 illustrates signed covariance between unintentional leak and ventilation used to determine a mouth leak, according to some implementations of the present disclosure;

FIG. 15 illustrates the feature separation for ventilation on levels of unintentional leak, according to some implementations of the present disclosure;

FIG. 16A illustrates negative epochs and positive epochs for each user before normalization, according to some implementations of the present disclosure;

FIG. 16B illustrates negative epochs and positive epochs for each user after normalization, according to some implementations of the present disclosure;

FIG. 17 illustrates the feature separation for unintentional leak variability, according to some implementations of the present disclosure;

FIG. 18A illustrates an example unintentional leak variance for high levels of unintentional leak in a user with mouth leak, according to some implementations of the present disclosure;

FIG. 18B illustrates an example unintentional leak variance for high levels of unintentional leak in a user without mouth leak, according to some implementations of the present disclosure;

FIG. 19 illustrates breath segmentation based on flow rate data, according to some implementations of the present disclosure;

FIG. 20A illustrates breath specific features calculated over a breath, according to some implementations of the present disclosure;

FIG. 20B illustrates additional breath specific features calculated over a portion of the breath, according to some implementations of the present disclosure;

FIG. 21 illustrates the ratio of breath area / frame area taken on flow rate data, with epoch $90^{th}$ percentile, according to some implementations of the present disclosure;

FIG. 22 illustrates the skewness taken on taken on flow rate data, with epoch mean, according to some implementations of the present disclosure;

FIG. 23 illustrates the skewness taken on derivative blower pressure, with epoch mean, according to some implementations of the present disclosure;

FIG. 24A acoustic power levels over a time period of no mask leak and a time period of mask leak, according to some implementations of the present disclosure;

FIG. 24B a comparative graphical representation of leak rate, flow rate, and mask pressure, over the time period of no mask leak and the time period of mask leak of FIG. 24A, according to some implementations of the present disclosure;

FIG. 25 illustrates a comparative graphical representation of maximum value of acoustic intensity, standard deviation of acoustic intensity, leak rate, flow rate, and mask pressure over a time period, according to some implementations of the present disclosure;

FIG. 26A acoustic power levels over a time period during which different types of leak occur, according to some implementations of the present disclosure; and

FIG. 26B comparative graphical representation of leak rate, flow rate, and mask pressure, over the time period of FIG. 26A, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, equivalents, and alternatives falling within the spirit and scope of the present disclosure as defined by the appended claims.

## DETAILED DESCRIPTION

Generally, healthy individuals breathe through their nose during sleep. Chronic mouth breathing can lead to increased congestion, dry mouth, bad breath, gingivitis, discomfort, and/or potentially nose bleeds.

There are many causes of mouth leak. Some people may breathe through their mouth at night if their nasal air passageway is obstructed (either completely blocked or partially blocked), which may be caused by congestion from allergies, a cold, or a sinus infection. Some people are predisposed to having an obstructed nasal air passageway, which may be caused by enlarged adenoids, enlarged tonsils, deviated septum, nasal polyps, or benign growths of tissue in the lining of the nose. Further, enlarged turbinates, the shape of the nose, and the shape and size of the jaw can contribute to an obstructed nasal air passageway.

Sleep apnea patients often also have an obstructed air passage way. For some people with sleep apnea, it may become a habit to sleep with their mouth open to accommodate their need for oxygen. In some instances, when sleep apnea patients begin CPAP therapy using a nasal mask or nasal pillows, they may inadvertently breathe through their mouth ("mouth leak"). For example, when the delta between the pressure in the mouth and the atmospheric pressure exceeds a threshold, the mouth (e.g., the lips) may pop open to normalize the pressure. The lips may close again on inhalation. This may not wake the patients, but will lead to dry mouth, dry lips, and discomfort when they wake. Some patients will not tolerate this for long, and are highly likely to stop their much needed therapy.

Some sleep apnea patients may have continuous mouth leak for at least a portion of the night, where their mouth remains open, and a continuous circuit is formed (air in through the nasal mask, and out through the mouth). Some patients will tolerate continuous mouth leak - even for 70% of the night - but they are unlikely to adhere to therapy long term and/or likely to only wear their mask earlier in the night (which is when the patients are in deep sleep rather than REM sleep). As such, for sleep apnea patients, mouth leak may reduce the effectiveness and/or comfort of therapy, which in turn leads to poorer outcomes and/or adherence to therapy.

Therefore, a need exists for a system that can detect if a user is mouth breathing, adjust appropriate settings on associated devices, and/or provide notifications to the user. The present disclosure is directed to such a system.

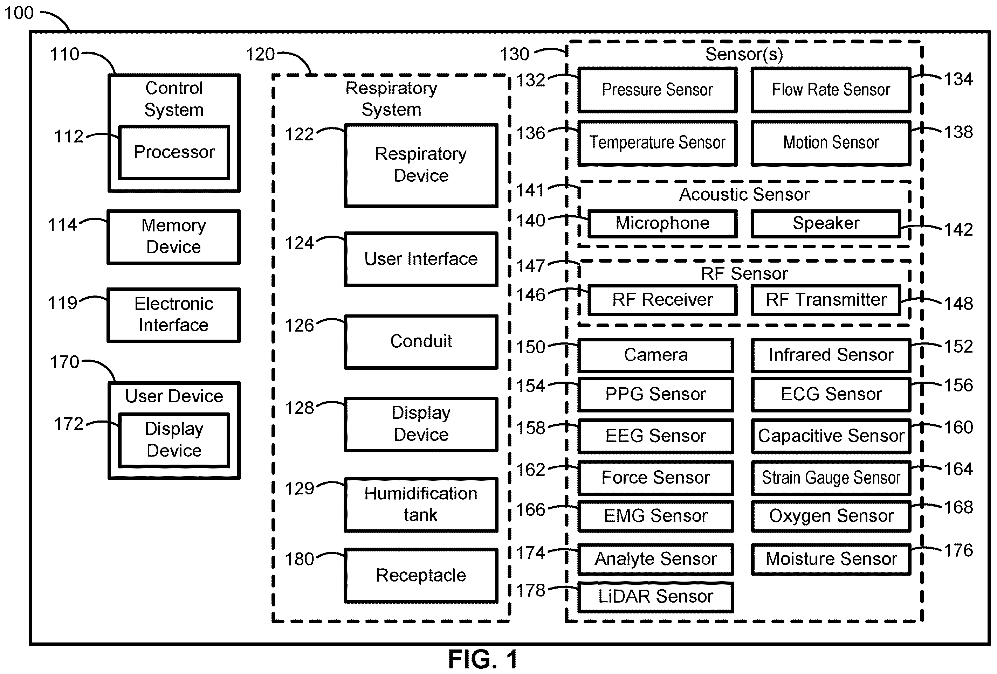

Referring to FIG. 1, a system 100, according to some implementations of the present disclosure, is illustrated. The system 100 includes a control system 110, a memory device 114, an electronic interface 119, one or more sensors 130, and one or more user devices 170. In some implementations, the system 100 further includes a respiratory system 120.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the user device 170, a portion (e.g., a housing) of the respiratory system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory device 122, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 (FIG. 1) stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a geographic location of the user, a relationship status, a family history of insomnia, an employment status of the user, an educational status of the user, a socioeconomic status of the user, or any combination thereof. The medical information can include, for example, including indicative of one or more medical conditions associated with the user, medication usage by the user, or both. The medical information data can further include a multiple sleep latency test (MSLT) test result or score and/or a Pittsburgh Sleep Quality Index (PSQI) score or value. The self-reported user feedback can include information indicative of a self-reported subjective therapy score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or audio data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a Wi-Fi communication protocol, a Bluetooth communication protocol, over a cellular network, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 can include a respiratory system 120 (also referred to as a respiratory therapy system). The respiratory system 120 can include a respiratory pressure therapy (RPT) device 122 (referred to herein as respiratory device 122), a user interface 124, a conduit 126 (also referred to as a tube or an air circuit), a display device 128, a humidification tank 129, a receptacle 180, or any combination thereof. In some implementations, the control system 110, the memory device 114, the display device 128, one or more of the sensors 130, and the humidification tank 129 are part of the respiratory device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory system 120 is generally used to treat individuals suffering from one or more sleep-related respiratory disorders (e.g., obstructive sleep apnea, central sleep apnea, or mixed sleep apnea).

The respiratory device 122 is generally used to generate pressurized air that is delivered to a user (e.g., using one or more motors that drive one or more compressors). In some implementations, the respiratory device 122 generates continuous constant air pressure that is delivered to the user. In other implementations, the respiratory device 122 generates two or more predetermined pressures (e.g., a first predetermined air pressure and a second predetermined air pressure). In still other implementations, the respiratory device 122 is configured to generate a variety of different air pressures within a predetermined range. For example, the respiratory device 122 can deliver at least about 6 $cmH_2O$, at least about 10 $cmH_2O$, at least about 20 $cmH_2O$, between about 6 $cmH_2O$ and about 10 $cmH_2O$, between about 7 $cmH_2O$ and about 12 $cmH_2O$, etc. The respiratory device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory device 122 to the user's airway to aid in preventing the airway from narrowing and/or collapsing during sleep. This may also increase the user's oxygen intake during sleep. Generally, the user interface 124 engages the user's face such that the pressurized air is delivered to the user's airway via the user's mouth, the user's nose, or both the user's mouth and nose. Together, the respiratory device 122, the user interface 124, and the conduit 126 form an air pathway fluidly coupled with an airway of the user. The pressurized air also increases the user's oxygen intake during sleep.

Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 $cmH_2O$ relative to ambient pressure. For other forms of therapy, such as the delivery of oxygen, the user interface may not include a seal sufficient to facilitate delivery to the airways of a supply of gas at a positive pressure of about 10 $cmH_2O$.

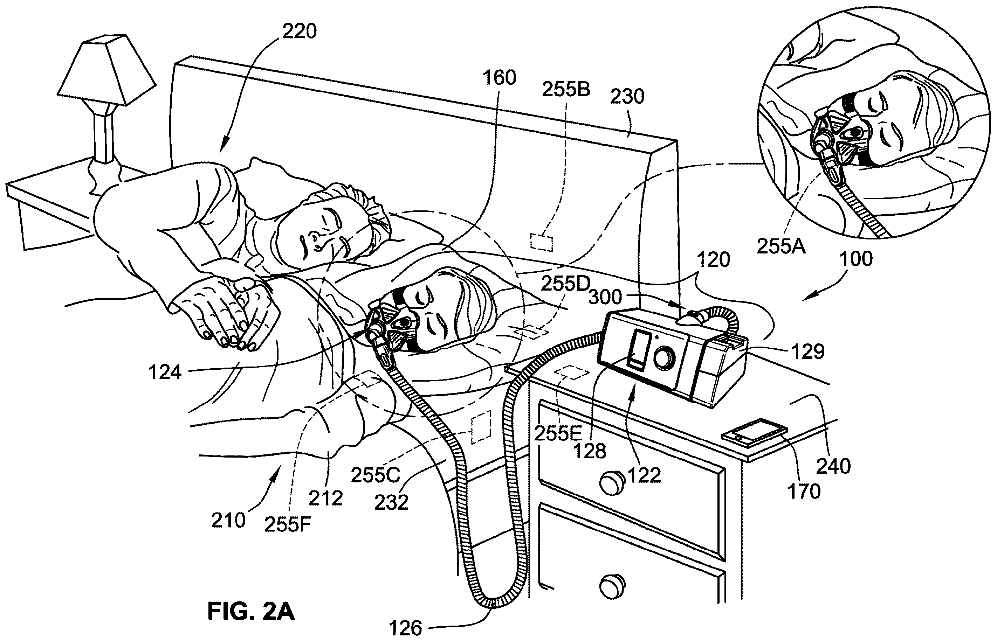

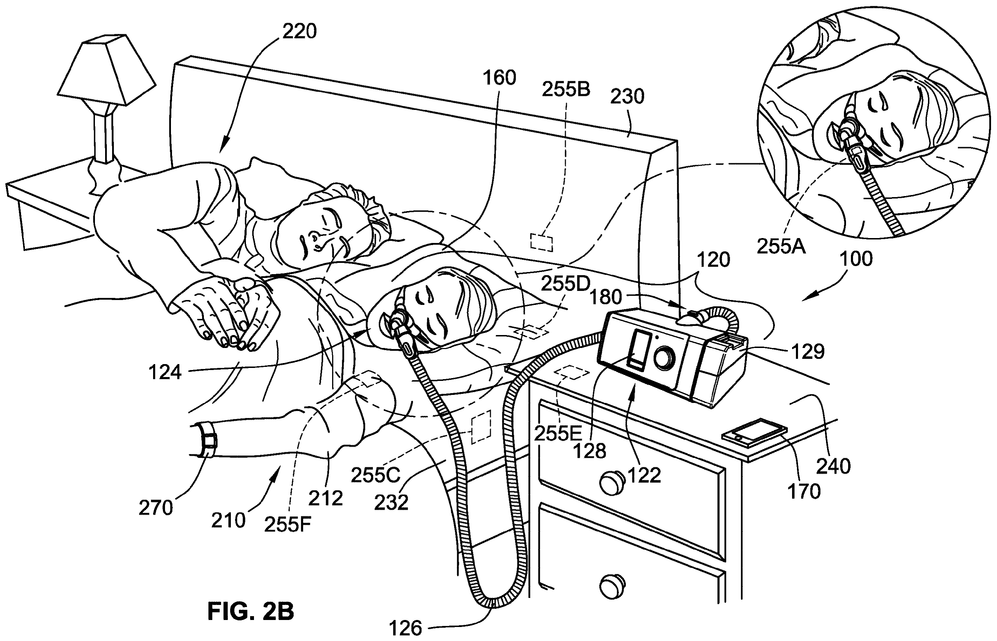

As shown in FIG. 2A, in some implementations, the user interface 124 is a facial mask (e.g., a full face mask) that covers the nose and mouth of the user. Alternatively, as shown in FIG. 2B, the user interface 124 is a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 can comprise a mouthpiece (e.g., a night guard mouthpiece molded to conform to the user's teeth, a mandibular repositioning device, etc.).

The conduit 126 (also referred to as an air circuit or tube) allows the flow of air between two components of a respiratory system 120, such as the respiratory device 122 and the user interface 124. In some implementations, there can be separate limbs of the conduit for inhalation and exhalation. In other implementations, a single limb conduit is used for both inhalation and exhalation.

One or more of the respiratory device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory device 122.

The display device 128 is generally used to display image(s) including still images, video images, or both and/or information regarding the respiratory device 122. For example, the display device 128 can provide information regarding the status of the respiratory device 122 (e.g., whether the respiratory device 122 is on/off, the pressure of the air being delivered by the respiratory device 122, the temperature of the air being delivered by the respiratory device 122, etc.) and/or other information (e.g., a sleep score or therapy score (also referred to as a myAir™ score, such as described in WO 2016/061629, which is hereby incorporated by reference herein in its entirety), the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory device 122.

The humidification tank 129 is coupled to or integrated in the respiratory device 122. The humidification tank 129 includes a reservoir of water that can be used to humidify the pressurized air delivered from the respiratory device 122. The respiratory device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself.

In some implementations, the system 100 can be used to deliver at least a portion of a substance from the receptacle 180 to the air pathway the user based at least in part on the physiological data, the sleep-related parameters, other data or information, or any combination thereof. Generally, modifying the delivery of the portion of the substance into the air pathway can include (i) initiating the delivery of the substance into the air pathway, (ii) ending the delivery of the portion of the substance into the air pathway, (iii) modifying an amount of the substance delivered into the air pathway, (iv) modifying a temporal characteristic of the delivery of the portion of the substance into the air pathway, (v) modifying a quantitative characteristic of the delivery of the portion of the substance into the air pathway, (vi) modifying any parameter associated with the delivery of the substance into the air pathway, or (vii) a combination of (i)-(vi).

Modifying the temporal characteristic of the delivery of the portion of the substance into the air pathway can include changing the rate at which the substance is delivered, starting and/or finishing at different times, continuing for different time periods, changing the time distribution or characteristics of the delivery, changing the amount distribution independently of the time distribution, etc. The independent time and amount variation ensures that, apart from varying the frequency of the release of the substance, one can vary the amount of substance released each time. In this manner, a number of different combination of release frequencies and release amounts (e.g., higher frequency but lower release amount, higher frequency and higher amount, lower frequency and higher amount, lower frequency and lower amount, etc.) can be achieved. Other modifications to the delivery of the portion of the substance into the air pathway can also be utilized.

The respiratory system 120 can be used, for example, a ventilator or as a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Still referring to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178 more generally, the one or more sensors 130 can include a combination and any number of each of the sensors described and/or shown herein.

As described herein, the system 100 generally can be used to generate physiological data associated with a user (e.g., a user of the respiratory system 120 shown in FIGS. 2A-2B) during a sleep session. The physiological data can be analyzed to generate one or more sleep-related parameters, which can include any parameter, measurement, etc. related to the user during the sleep session. The one or more sleep-related parameters that can be determined for the user 210 during the sleep session include, for example, an Apnea-Hypopnea Index (AHI) score, a sleep score, a flow signal, a respiration signal, a respiration rate, an inspiration amplitude, an expiration amplitude, an inspiration-expiration ratio, a number of events per hour, a pattern of events, a stage, pressure settings of the respiratory device 122, a heart rate, a heart rate variability, movement of the user 210, temperature, EEG activity, EMG activity, arousal, snoring, choking, coughing, whistling, wheezing, or any combination thereof.

The one or more sensors 130 can be used to generate, for example, physiological data, audio data, or both. Physiological data generated by one or more of the sensors 130 can be used by the control system 110 to determine a sleep-wake signal associated with the user 210 during the sleep session and one or more sleep-related parameters. The sleep-wake signal can be indicative of one or more sleep states and/or one or more sleep stages, including wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), or any combination thereof.

The sleep-wake signal can also be timestamped to determine a time that the user enters the bed, a time that the user exits the bed, a time that the user attempts to fall asleep, etc. The sleep-wake signal can be measured by the one or more sensors 130 during the sleep session at a predetermined sampling rate, such as, for example, one sample per second, one sample per 30 seconds, one sample per minute, etc. In some implementations, the sleep-wake signal can also be indicative of a respiration signal, a respiration rate, an inspiration amplitude, an expiration amplitude, an inspiration-expiration ratio, a number of events per hour, a pattern of events, pressure settings of the respiratory device 122, or any combination thereof during the sleep session. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof. The one or more sleep-related parameters that can be determined for the user during the sleep session based on the sleep-wake signal include, for example, a total time in bed, a total sleep time, a sleep onset latency, a wake-after-sleep-onset parameter, a sleep efficiency, a fragmentation index, or any combination thereof.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration ratio, a number of events per hour, a pattern of events, pressure settings of the respiratory device 122, or any combination thereof. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof.

Generally, the sleep session includes any point in time after the user 210 has laid or sat down in the bed 230 (or another area or object on which they intend to sleep), and has turned on the respiratory device 122 and donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory device 122, and gets out of bed 230. In some implementations, the sleep session can include additional periods of time, or can be limited to only some of the above-disclosed time periods. For example, the sleep session can be defined to encompass a period of time beginning when the respiratory device 122 begins supplying the pressurized air to the airway or the user 210, ending when the respiratory device 122 stops supplying the pressurized air to the airway of the user 210, and including some or all of the time points in between, when the user 210 is asleep or awake.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory system 120 and/or ambient pressure. In such implementations, the pressure sensor 132 can be coupled to or integrated in the respiratory device 122. The pressure sensor 132 can be, for example, a capacitive sensor, an electromagnetic sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory device 122, an air flow rate through the conduit 126, an air flow rate through the user interface 124, or any combination thereof. In such implementations, the flow rate sensor 134 can be coupled to or integrated in the respiratory device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIGS. 2A-2B), a skin temperature of the user 210, a temperature of the air flowing from the respiratory device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user 210 during the sleep session, and/or detect movement of any of the components of the respiratory system 120, such as the respiratory device 122, the user interface 124, or the conduit 126. The motion sensor 138 can include one or more inertial sensors, such as accelerometers, gyroscopes, and magnetometers. In some implementations, the motion sensor 138 alternatively or additionally generates one or more signals representing bodily movement of the user, from which may be obtained a signal representing a sleep state of the user; for example, via a respiratory movement of the user. In some implementations, the motion data from the motion sensor 138 can be used in conjunction with additional data from another sensor 130 to determine the sleep state of the user.

The microphone 140 outputs sound data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the respiratory device 122, the user interface 124, the conduit 126, or the user device 170. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones.

The speaker 142 outputs sound waves that are audible to a user of the system 100 (e.g., the user 210 of FIGS. 2A-2B). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the respiratory device 122, the user interface 124, the conduit 126, or the external device 170.

The microphone 140 and the speaker 142 can be used as separate devices. In some implementations, the microphone 140 and the speaker 142 can be combined into an acoustic sensor 141, as described in, for example, WO 2018/050913 and WO 2020/104465, each of which is hereby incorporated by reference herein in its entirety. In such implementations, the speaker 142 generates or emits sound waves at a predetermined interval and/or frequency and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIGS. 2A-2B). Based at least in part on the data from the microphone 140 and/or the speaker 142, the control system 110 can determine a location of the user 210 (FIGS. 2A-2B) and/or one or more of the sleep-related parameters (e.g., a mouth leak status) described in herein, such as, for example, a respiration signal, a respiration rate, an inspiration amplitude, an expiration amplitude, an inspiration-expiration ratio, a number of events per hour, a pattern of events, a sleep state, pressure settings of the respiratory device 122, or any combination thereof. In this context, a sonar sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO2018/050913 and WO 2020/104465 mentioned above.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIGS. 2A-2B) and/or one or more of the sleep-related parameters described herein. An RF receiver (either the RF receiver 146 and the RF transmitter 148 or another RF pair) can also be used for wireless communication between the control system 110, the respiratory device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147. In some such implementations, the RF sensor 147 includes a control circuit. The specific format of the RF communication could be Wi-Fi, Bluetooth, or the like.

In some implementations, the RF sensor 147 is a part of a mesh system. One example of a mesh system is a Wi-Fi mesh system, which can include mesh nodes, mesh router(s), and mesh gateway(s), each of which can be mobile/movable or fixed. In such implementations, the Wi-Fi mesh system includes a Wi-Fi router and/or a Wi-Fi controller and one or more satellites (e.g., access points), each of which include an RF sensor that the is the same as, or similar to, the RF sensor 147. The Wi-Fi router and satellites continuously communicate with one another using Wi-Fi signals. The Wi-Fi mesh system can be used to generate motion data based on changes in the Wi-Fi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or any combination thereof) that can be stored in the memory device 114. The image data from the camera 150 can be used by the control system 110 to determine one or more of the sleep-related parameters described herein, such as, for example, one or more events (e.g., periodic limb movement or restless leg syndrome), a respiration signal, a respiration rate, an inspiration amplitude, an expiration amplitude, an inspiration-expiration ratio, a number of events per hour, a pattern of events, a sleep state, a sleep stage, or any combination thereof. Further, the image data from the camera 150 can be used to, for example, identify a location of the user, to determine chest movement of the user 210, to determine air flow of the mouth and/or nose of the user 210, to determine a time when the user 210 enters the bed 230, and to determine a time when the user 210 exits the bed 230.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIGS. 2A-2B) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210 (FIGS. 2A-2B). In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210.

The data output by the analyte sensor 174 can be stored in the memory device 114 and used by the control system 110 to determine the identity and concentration of any analytes in the user 210's breath. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user 210's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the processor 112 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory device 122, etc.). Thus, in some implementations, the moisture sensor 176 can be positioned in the user interface 124 or in the conduit 126 to monitor the humidity of the pressurized air from the respiratory device 122. In other implementations, the moisture sensor 176 is placed near any area where moisture levels need to be monitored. The moisture sensor 176 can also be used to monitor the humidity of the ambient environment surrounding the user 210, for example the air inside the user 210's bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

In some implementations, the one or more sensors 130 also include a galvanic skin response (GSR) sensor, a blood flow sensor, a respiration sensor, a pulse sensor, a sphygmomanometer sensor, an oximetry sensor, a sonar sensor, a RADAR sensor, a blood glucose sensor, a color sensor, a pH sensor, an air quality sensor, a tilt sensor, a rain sensor, a soil moisture sensor, a water flow sensor, an alcohol sensor, or any combination thereof.

While shown separately in FIG. 1, a combination of the one or more sensors 130 can be integrated in and/or coupled to any one or more of the components of the system 100, including the respiratory device 122, the user interface 124, the conduit 126, the humidification tank 129, the control system 110, the user device 170, or any combination thereof. For example, the acoustic sensor 141 and/or the RF sensor 147 can be integrated in and/or coupled to the user device 170. In such implementations, the user device 170 can be considered a secondary device that generates additional or secondary data for use by the system 100 (e.g., the control system 110) according to some aspects of the present disclosure. In some implementations, at least one of the one or more sensors 130 is not coupled to the respiratory device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, a sleep state, an apnea-hypopnea index (AHI), or any combination thereof. The one or more events can include snoring, apneas, central apneas, obstructive apneas, mixed apneas, hypopneas, a mask leak, a cough, a restless leg, a sleeping disorder, choking, an increased heart rate, labored breathing, an asthma attack, an epileptic episode, a seizure, increased blood pressure, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, or from other types of data.

The user device 170 (FIG. 1) includes a display device 128. The user device 170 can be, for example, a mobile device such as a smart phone, a tablet, a gaming console, a smart watch, a laptop, or the like. Alternatively, the user device 170 can be an external sensing system, a television (e.g., a smart television) or another smart home device (e.g., a smart speaker(s) such as Google Home, Amazon Echo, Alexa etc.). In some implementations, the user device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices can be used by and/or included in the system 100.

While the control system 110 and the memory device 114 are described and shown in FIG. 1 as being a separate and distinct component of the system 100, in some implementations, the control system 110 and/or the memory device 114 are integrated in the user device 170 and/or the respiratory device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for generating physiological data and determining a recommended bedtime for the user according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory system 120, at least one of the one or more sensors 130, and the user device 170. Thus, various systems for determining a recommended bedtime for the user can be formed using any portion or portions of the components shown and described herein and/or in combination with one or more other components.

Generally, a user who is prescribed usage of a respiratory system will tend to experience higher quality sleep and less fatigue during the day after using the respiratory system 120 during the sleep compared to not using the respiratory system 120 (especially when the user suffers from sleep apnea or other sleep related disorders). However, many users do not conform to their prescribed usage because the user interface 124 is uncomfortable or cumbersome, or due to other side effects (e.g., dry mouth, dry lips, dry throat, discomfort, etc.). Users are more likely to fail to use the respiratory system 120 as prescribed (or discontinue usage altogether) if they fail to perceive that they are experiencing any benefits (e.g., less fatigue during the day).

However, the side effects and/or the lack of improvement in sleep quality may be due to mouth leak rather than a lack of efficacy to the treatment. Thus, it is advantageous to determine a mouth leak status for the user, and communicate the mouth leak status to the user to aid the user in obtaining higher quality sleep, so that the user does not discontinue or reduce their usage of the respiratory system 120 due to a perceived lack of benefit(s).

Referring generally to FIGS. 2A-2B, a portion of the system 100 (FIG. 1), according to some implementations, is illustrated. A user 210 of the respiratory system 120 and a bed partner 220 are located in a bed 230 and are laying on a mattress 232. The user interface 124 (e.g., a full facial mask in FIG. 2A or a nasal mask in FIG. 2B) can be worn by the user 210 during a sleep session. The user interface 124 is fluidly coupled and/or connected to the respiratory device 122 via the conduit 126. In turn, the respiratory device 122 delivers pressurized air to the user 210 via the conduit 126 and the user interface 124 to increase the air pressure in the throat of the user 210 to aid in preventing the airway from closing and/or narrowing during sleep. The respiratory device 122 can be positioned on a nightstand 240 that is directly adjacent to the bed 230 as shown in FIG. 2A, or more generally, on any surface or structure that is generally adjacent to the bed 230 and/or the user 210.

In some implementations, the control system 110, the memory 214, any of the one or more sensors 130, or any combination thereof can be located on and/or in any surface and/or structure that is generally adjacent to the bed 230 and/or the user 210. For example, in some implementations, at least one of the one or more sensors 130 can be located at a first position 255A on and/or in one or more components of the respiratory system 120 adjacent to the bed 230 and/or the user 210. The one or more sensors 130 can be coupled to the respiratory system 120, the user interface 124, the conduit 126, the display device 128, the humidification tank 129, or any combination thereof.

Alternatively or additionally, at least one of the one or more sensors 130 can be located at a second position 255B on and/or in the bed 230 (e.g., the one or more sensors 130 are coupled to and/or integrated in the bed 230). Further, alternatively or additionally, at least one of the one or more sensors 130 can be located at a third position 255C on and/or in the mattress 232 that is adjacent to the bed 230 and/or the user 210 (e.g., the one or more sensors 130 are coupled to and/or integrated in the mattress 232). Alternatively or additionally, at least one of the one or more sensors 130 can be located at a fourth position 255D on and/or in a pillow that is generally adjacent to the bed 230 and/or the user 210.

Alternatively or additionally, at least one of the one or more sensors 130 can be located at a fifth position 255E on and/or in the nightstand 240 that is generally adjacent to the bed 230 and/or the user 210. Alternatively or additionally, at least one of the one or more sensors 130 can be located at a sixth position 255F such that the at least one of the one or more sensors 130 are coupled to and/or positioned on the user 215 (e.g., the one or more sensors 130 are embedded in or coupled to fabric, clothing 212, and/or a smart device 270 worn by the user 210). More generally, at least one of the one or more sensors 130 can be positioned at any suitable location relative to the user 210 such that the one or more sensors 130 can generate sensor data associated with the user 210.

In some implementations, a primary sensor, such as the microphone 140, is configured to generate acoustic data associated with the user 210 during a sleep session. For example, one or more microphones (the same as, or similar to, the microphone 140 of FIG. 1) can be integrated in and/or coupled to (i) a circuit board of the respiratory device 122, (ii) the conduit 126, (iii) a connector between components of the respiratory system 120, (iv) the user interface 124, (v) a headgear (e.g., straps) associated with the user interface, or (vi) any combination thereof. In some implementations, the microphone is in fluid communication and/or acoustic communication with the airflow pathway (e.g., an air pathway fluidly coupled with an airway of the user). For example, in some implementations, the microphone is positioned on a printed circuit board connected via duct to the airflow pathway.

Additionally or alternatively, one or more microphones (the same as, or similar to, the microphone 140 of FIG. 1) can be integrated in and/or coupled to a co-located smart device, such as the user device 170, a TV, a watch (e.g., a mechanical watch or the smart device 270), a pendant, the mattress 232, the bed 230, beddings positioned on the bed 230, the pillow, a speaker (e.g., the speaker 142 of FIG. 1), a radio, a tablet, a waterless humidifier, or any combination thereof.

Additionally or alternatively, in some implementations, one or more microphones (the same as, or similar to, the microphone 140 of FIG. 1) can be remote from the system 100 (FIG. 1) and/or the user 210 (FIGS. 2A-2B), so long as there is an air passage allowing acoustic signals to travel to the one or more microphones. For example, the one or more microphones can be in a different room from the room containing the system 100.

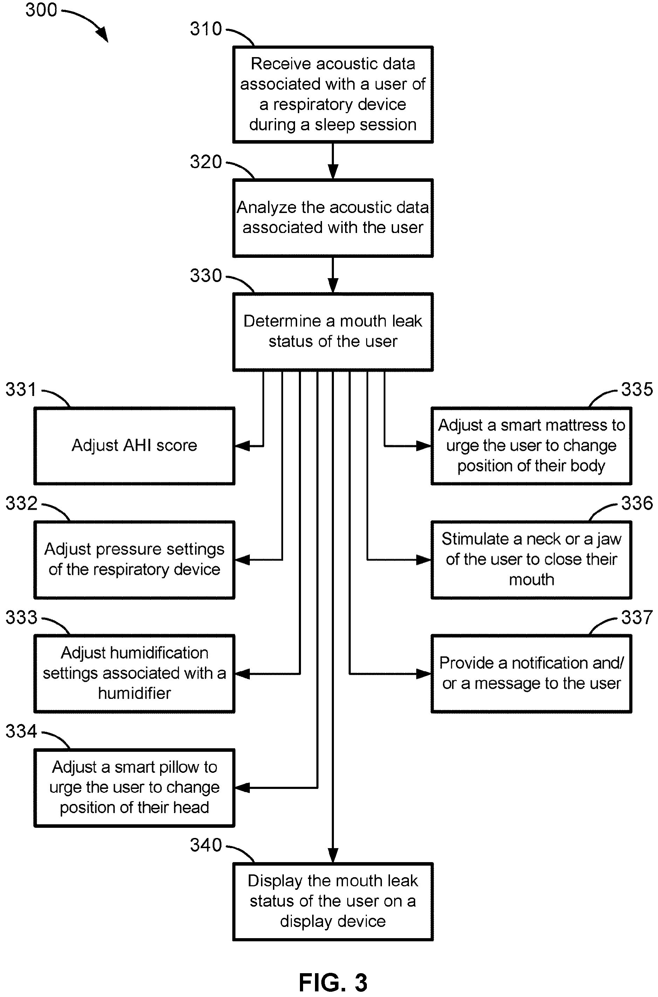

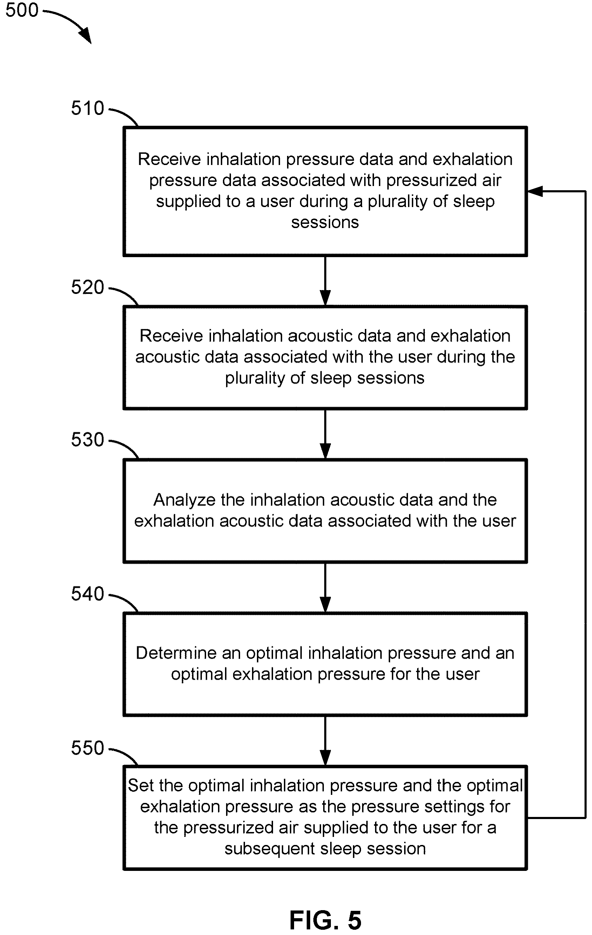

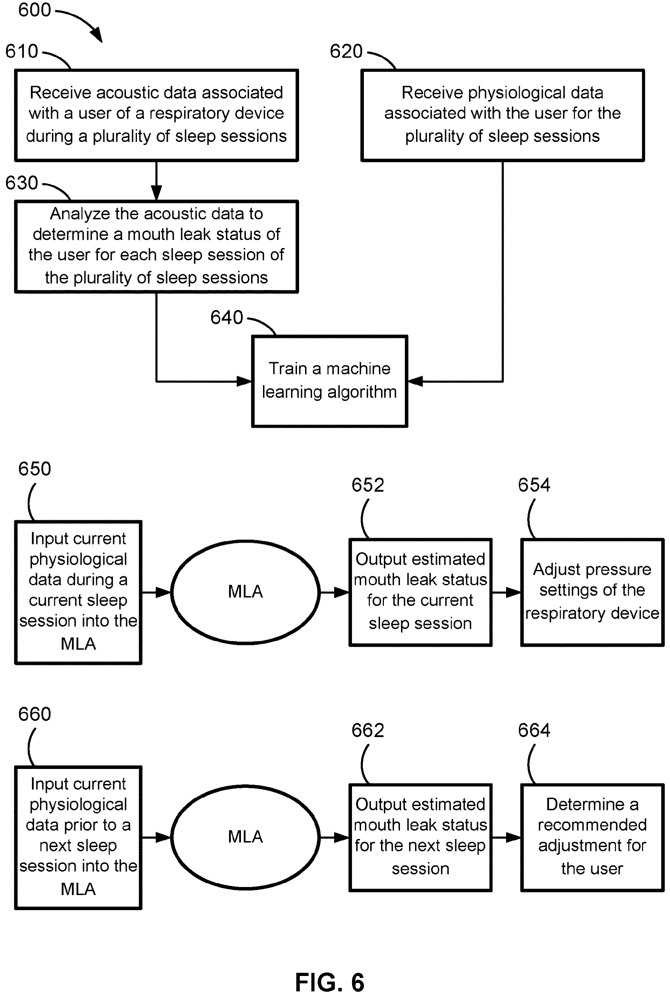

Based at least in part on an analysis of the acoustic data, a mouth leak status can be determined. The mouth leak status is indicative of air leaking from a mouth of the user (e.g., the mouth leak as described herein). Additionally, in some implementations, the determining the mouth leak status includes distinguishing mouth leak from mask leak. In some implementations, the mouth leak status is determined using one or more steps of methods 300 (FIG. 3), 500 (FIG. 5), and 600 (FIG. 6) of the present disclosure.

Referring to FIG. 3, a method 300 for determining a mouth leak status for a user is illustrated. One or more steps of the method 300 can be implemented using any element or aspect of the system 100 (FIGS. 1 and 2A-2B) described herein.

Step 310 of the method 300 includes generating or obtaining acoustic data associated with a user during at least a portion of a sleep session. For example, step 310 can include generating or obtaining acoustic data during the sleep session using at least of the one or more sensors 130 (FIG. 1). In some implementations, the acoustic data is generated using one or more microphones (such as the microphone 140 described above). In some implementations, at least one of the one or more microphones is coupled to or integrated in the user interface 124. Additionally or alternatively, in some implementations, the acoustic data is generated using an external microphone that is not a component of the system 100. In some implementations, the acoustic data is generated using the acoustic sensor 141 and/or the RF sensor 147 described above, which are coupled to or integrated in the respiratory system 120 (FIG. 1). Information describing the acoustic data generated or obtained during step 310 can be stored in the memory device 114 (FIG. 1).

Step 310 can include generating acoustic data (via a primary sensor such as the microphone 140) during a segment of the sleep session, during the entirety of the sleep session, or across multiple segments of the first sleep session. For example, step 310 can include generating acoustic data continuously, or only based on secondary sensor data generated by a secondary sensor. For example, a temperature sensor (e.g., the temperature sensor 136) and/or an analyte sensor (e.g., the analyte sensor 174), may be positioned close to the mouth of the user to directly detect mouth breathing.

In some implementations, one or more secondary sensors may be used in addition to the primary sensor to confirm the mouth leak status. In some such implementations, the one or more secondary sensors include: a flow rate sensor (e.g., the flow rate sensor 134 of the system 100), a temperature sensor (e.g., the temperature sensor 136 of the system 100), a camera (e.g., the camera 150 of the system 100), a vane sensor (VAF), a hot wire sensor (MAF), a cold wire, a laminar flow sensor, an ultrasonic sensor, an inertial sensor, or any combination thereof.

The flow rate sensor 134 can be used to generate flow data (in the form of flow rate data) associated with the user 210 (FIGS. 2A-2B) of the respiratory device 122 during the sleep session. Examples of flow rate sensors (such as, for example, the flow rate sensor 134) are described in International Publication No. WO 2012/012835, which is hereby incorporated by reference herein in its entirety. In some implementations, the flow rate sensor 134 is configured to measure a vent flow (e.g., intentional "leak"), an unintentional leak (e.g., mouth leak and/or mask leak), a patient flow (e.g., air into and/or out of lungs), or any combination thereof. In some implementations, the flow rate data can be analyzed to determine cardiogenic oscillations of the user.

The camera 150 can be used to generate image data associated with the user during the sleep session. As described herein, the camera can be configured to detect a facial anatomy (e.g., shape (e.g. open, partially open, or closed) and/or dimension of the mouth, the nostrils), a respiration signal, a respiration rate, an inspiration amplitude, an expiration amplitude, an inspiration-expiration ratio, a number of events per hour, a pattern of events, a sleep state, a sleep stage, or any combination thereof.

Therefore, in some implementations, step 310 of the method 300 further includes generating or obtaining physiological data associated with the user during the sleep session. For example, step 310 can include generating or obtaining physiological data during the sleep session using at least of the one or more sensors 130 (FIG. 1). Information describing the physiological data generated during step 310 can be stored in the memory device 114 (FIG. 1).

In some implementations, a single sensor can generate both the acoustic data and the physiological data. Alternatively, the acoustic data is generated using a first one of the sensors 130 and the physiological data is generated using a second of the sensors 130 that is separate and distinct from the first sensor. In some implementations, the first sensor and the second sensor can be different types of sensors (e.g., the first sensor is a microphone that is the same as, or similar to, the microphone 140, and the second sensor is a motion sensor that is the same as, or similar to, the motion sensor 138). Alternatively, in some implementations, the first sensor and the second sensor can be two of the same sensors (e.g., two microphones that are the same as, or similar to, the microphone 140). For example, in some implementations, a first microphone is an integrated microphone coupled to a conduit of the respiratory device. The second microphone is an external microphone.

Step 320 of the method 300 includes analyzing the acoustic data associated with the user. The control system 110 can analyze the acoustic data stored in the memory device 114 to determine the mouth leak status. In some implementations, for analyzing the acoustic data, the acoustic data (step 310) is compared with predetermined data indicative of a negative mouth leak status. The predetermined data can include simulated data, historical data, or both.

For example, in some implementations, acoustic data indicative of intentional leak of the mask can be estimated for any given mask. The type of mask can be identified using, for example, a cepstrum analysis described herein. The acoustic data as measured by the microphone 140 is compared with the estimated intentional leak. If the respiratory system is a closed system (e.g., no mouth leak), there should be a reasonable match. However, if the system is "open" due to, for example, mouth leak, the acoustic data deviates (above a predetermined threshold) from the estimated intentional leak.

In some implementations, the acoustic data (step 310) includes reflected sound waves received by a microphone (e.g., the microphone 140 of the system 100) that are transmitted from a speaker (e.g., the speaker 142 of the system 100, or an external speaker). The reflected sound waves are indicative of shapes and dimensions of the components in the sound waves' path(s). Additionally or alternatively, the acoustic data includes sound(s) from the user that is indicative of one or more sleep-related parameters (e.g., breathing through the nose, breathing through the mouth, snoring, sniffling).

For example, the acoustic data (step 310) can include data generated by the microphone 140. The speaker 142 generates a sound. The sound can travel through the humidification tank 129, along a first connection, along the conduit 126, via a second connection, via a waterless humidifier (if fitted), to one or more mask cavities (e.g., nostrils and/or mouth), to the user's respiratory system (including nose and/or mouth, airway(s), lungs, etc.). For each change in the path (e.g., a cavity, a junction, a change in shape), a reflection at that point based on speed of sound is seen. The different types and distances of reflection(s) can be used to define a type and/or a model of user interface 124.

The further reflections can be used to define aspects of the user's respiratory system (including if one or both nostrils are being used, and/or if the mouth being used to breathe). These reflections change as the user breathes in and out, and further change on exhalation if the mouth pops open. In some implementations, a reduction in the mask cavity response can be seen in the reflections when mouth leak occurs. For example, if the user is having a mouth leak, the expected echo signal (such as might be detected at other times of the night when the mouth is closed) comes out the mouth rather than back down the conduit 126 to the microphone 140.

In some implementations, a cepstrum analysis is implemented to analyze the acoustic data. Cepstrum is a "quefrency" domain, which is also known as the spectrum of the log of a time domain waveform. For example, a cepstrum may be considered the inverse Fourier Transform of the log spectrum of the forward Fourier Transform of the decibel spectrum, etc. The operation essentially can convert a convolution of an impulse response function (IRF) and a sound source into an addition operation so that the sound source may then be more easily accounted for or removed so as to isolate data of the IRF for analysis. Techniques of cepstrum analysis are described in detail in a scientific paper entitled "The Cepstrum: A Guide to Processing" (Childers et al, Proceedings of the IEEE, Vol. 65, No. 10, Oct 1977) and Randall RB, Frequency Analysis, Copenhagen: Bruel & Kjaer, p. 344 (1977, revised ed. 1987).

Such a method may be understood in terms of the property of convolution. The convolution of f and g can be written as f * *g*. This operation can be the integral of the product of the two functions (f and g) after one is reversed and shifted. As such, it is a type of integral transform as Equation 1, as follows:

$$(f * g)(t) \stackrel{\text{def}}{=} \int_{-\infty}^{\infty} f(\tau) \cdot g(t - \tau) d\tau \qquad \text{Eq. 1}$$

While the symbol t is used above, it need not represent the time domain. But, in that context, the convolution formula can be described as a weighted average of the function $f(\tau)$ at the moment t where the weighting is given by $g(-\tau)$ simply shifted by amount t. As t changes, the weighting function emphasizes different parts of the input function.

More generally, if f and g are complex-valued functions on $R^d$, then their convolution may be defined as the integral of Equation 2:

$$(f * g)(x) = \int_{R^d} f(y)g(x - y)dy = \int_{R^d} f(x - y)g(y)dy \qquad \text{Eq. 2}$$

A mathematical model that can relate an acoustic system output to the input for a time-invariant linear system, such as one involving conduits of a respiratory treatment apparatus, (which may include some human or other unknown part of the system) can be based on this convolution. The output measured at a microphone of the system may be considered as the input noise "convolved" with the system Impulse Response Function (IRF) as a function of time (t), as shown in Equation 3:

$$y(t) = s_1(t) * h_1(t) \qquad \text{Eq. 3}$$

where * denotes the convolution function; $y(t)$ is the signal measured at the sound sensor; $S_1(t)$ is the sound or noise source such, as a noise or sound created in or by a flow generator of a respiratory treatment apparatus; and $h_1(t)$ is the system IRF from the noise or sound source to the sound sensor. The Impulse Response Function (IRF) is the system response to a unit impulse input.

Conversion of Equation 3 into the frequency domain by means of the Fourier Transform of the measured sound data (e.g., a discrete Fourier Transform ("DFT") or a fast Fourier transform ("FFT") and considering the Convolution Theorem, Equation 4 is produced:

$$y(t) = s_1(t) * h_1(t) \xrightarrow{\text{Fourier Transform}} Y(f) = S_1(f)H_1(f) \qquad \text{Eq. 4}$$

where $Y(f)$ is the Fourier Transform of $y(t)$; $S_1(f)$ is the Fourier Transform of $s_1(t)$; and $H_1(f)$ is the Fourier Transform of $h_1(t)$. In such a case, convolution in the time domain becomes a multiplication in the frequency domain.

A logarithm of Equation 4 may be applied so that the multiplication is converted into an addition, resulting in Equation 5:

$$Log\{Y(f)\} = Log\{S_1(f)H_1(f)\} = Log\{S_1(f)\} + Log\{H_1(f)\} \qquad \text{Eq. 5}$$

Equation 5 may then be converted back into the time domain, by an Inverse Fourier Transform (IFT) (e.g., an inverse DFT or inverse FFT), which results in a complex cepstrum ($K(\tau)$) (complex because one can work from the complex spectrum) - the inverse Fourier Transform of the logarithm of the spectrum; Equation 6.

$$K(\tau) = IFT[Log\{S_1(f)\} + Log\{H_1(f)\}] \qquad \text{Eq. 6}$$

where "$\tau$" is a real valued variable known as quefrency, with units measured in seconds. From this, the effects that are convolutive in the time domain become additive in the logarithm of the spectrum, and remain so in the cepstrum.

Consideration of the data from a cepstrum analysis, such as examining the data values of the quefrency, may provide information about the system. For example, by comparing cepstrum data of a system from a prior or known baseline of cepstrum data for the system, the comparison, such as a difference, can be used to recognize differences or similarities in the system that may then be used to implement varying functions or purposes disclosed herein. The following disclosure can utilize the methodologies of such an analysis, as herein explained, to implement the detection of cardiac output.

Therefore, in some implementations, analysis of the acoustic data using cepstrum can be used to measure the cross-sectional area, and change(s) in the cross-sectional area of the user interface 124, the nasal passages, the estimated dimensions of sinuses. The changes in the nasal passages and the estimated dimensions of the sinuses may be indicative of inflammation and/or congestion.

In some implementations, direct spectral methods can be implemented to analyze the acoustic data. Some examples of direct spectral methods include processing discrete Fourier transform (DFT), fast Fourier transform (FFT) with a sliding window, short time Fourier transform (STFT), wavelets, wavelet-based cepstrum calculation, deep neural networks (e.g., using imaging methods applied to spectrograms), Hilbert-Huang transform (HHT), empirical mode decomposition (EMD), blind source separation (BSS), Kalman filters, or any combination thereof. In some implementations, cepstral coefficients (CCs) such as mel-frequency cepstral coefficients (MFCCs) may be used, for example, by treating the acoustic data analysis as a speech recognition problem and using a machine learning / classification system.

For example, in some implementations, the acoustic data (step 310) can be analyzed to detect congestion and/or occlusion of one or both nasal passages (due to, for example, an illness or allergy). In some implementations, the acoustic data (step 310) can be analyzed to measure parameters associated with the respiratory anatomy of the user. For example, as discussed herein, certain abnormalities of the respiratory anatomy are associated with an obstructed nasal air passageway, such as enlarged adenoids, enlarged tonsils, deviated septum, nasal polyps, or benign growths of tissue in the lining of the nose. Further, enlarged turbinates, the shape of the nose, and the shape and size of the jaw can also contribute to an obstructed nasal air passageway. In some implementations, the acoustic data (step 310) can be analyzed to measure dimensions of the nasal passages using the acoustic data, and/or any changes in the dimensions.

In some implementations, the acoustic data can be processed to determine cardiogenic oscillations due to, for example, heart beats in the acoustic signal. Analysis of the cardiogenic oscillations can in turn, be processed to determine the mouth leak status. The characteristics of the cardiogenic oscillations may be different on inhalation and/or exhalation when the mouth is open versus closed. A change in heart rate is also seen due to the micro arousal (e.g., brief awakening) during mouth leak, which can be indicative of the physiological impact of the brain detecting the mouth leak. In some implementations, weaker or no cardiogenic oscillations is indicative of mouth leak. For example, the cardiogenic oscillations have a reduced fidelity when there is mouth leak.

In some implementations, for analyzing the acoustic data, the acoustic data (step 310) is processed to identify a plurality of features. The plurality of features can be indicative of the mouth leak status, and/or further processed to determine the mouth leak status. For example, the plurality of features can include: one or more changes in a spectral signature of an acoustic signal, one or more changes in a frequency of the sound waves, one or more changes in an amplitude of the sound waves, mel-frequency cepstral coefficients (MFCCs), a spectral flux, a spectral centroid, a harmonic product spectrum, a spectral spread, spectral autocorrelation coefficients, a spectral kurtosis, a linear predictive coding (LPC), or any combination thereof.

Additionally or alternatively, the plurality of features can include: a root mean square (RMS), zero-crossings, an envelope, a pitch, or any combination thereof, based on an auto-correlation. Additionally or alternatively, the plurality of features can include: a change in echo reflected signal shape (e.g., a reduction in amplitude and/or an apparent shift of shape as the nature of air circuit changes).

In some implementations, a band pass filtered white noise source generates or emits sound waves at a predetermined interval and a microphone (e.g., the microphone 140 of FIG. 1) detects the reflections of the emitted sound waves from the white noise source. The nature of the signature could be synchronized with expiration, and separable from the typical sound of expiration when the mouth is closed (e.g., if the user is using a nasal mask). In some such implementations, the plurality of features can include the signature synchronized with the expiration.

Step 330 of the method 300 includes determining a mouth leak status for the user for the sleep session based at least in part on the acoustic data, the physiological data, or both.

In some implementations, the acoustic data (step 310) can be analyzed (step 320; independently or in conjunction with the physiological data) to determine a probability of mouth leak and/or a probability relating to a severity of mouth leak. In some implementations, the physiological data can be analyzed (independently or in conjunction with the acoustic data) to determine a probability of mouth leak and/or a probability relating to a severity of mouth leak. For example, snoring, sleep position, head position, sleep stage, congestion, pillow configuration, alcohol consumption, body temperature, allergens in ambient air, body weight, body composition, neck size, gender, being a new user, type of mask, or any combination thereof can contribute to either or both of the probabilities.

In some implementations, the mouth leak status is determined, at step 330, based on data generated by two or more separate and distinct sensors. Having two or more sensors can increase the fidelity of the determination of the mouth leak status. For example, a system can include a microphone (that is the same as, or similar to, the microphone 140 of the system 100) and a flow rate sensor (that is the same as, or similar to, the flow rate sensor 134 of the system 100). Acoustic data associated with a user of a respiratory device (e.g., the user 210 of the respiratory device 122) is received from the microphone (e.g., step 310). In addition, flow data associated with the user of the respiratory device is received from the flow rate sensor. The acoustic data is analyzed (e.g., step 320). The flow data is also analyzed (e.g., one or more steps disclosed in WO 2012/012835 incorporated by reference herein). The mouth leak status is then determined based, at least in part, on both the analysis of the acoustic data and the analysis of the flow data.

In some implementations, step 330 includes using a machine learning algorithm to determine the mouth leak status for the user. For example, step 330 can include using neural networks (e.g., shallow or deep approaches) to determine the mouth leak status. Step 330 can include using supervised machine learning algorithms/techniques and/or unsupervised machine learning algorithms/techniques. For example, in some implementations, the acoustic data (step 310) is processed using the machine learning algorithm to output the mouth leak status for the user.

Optionally, in some implementations, step 340 of the method 300 includes displaying the mouth leak status of the user on a display device (e.g., the display device 172 of the user device 170 and/or the display device 128 of the respiratory system 120).

In some implementations, the method 300 further includes step 331, where an AHI number (or a therapy number such as a MyAir™ number) and/or an AHI score (or a therapy score such as a MyAir™ score) is calculated and/or modified based at least in part on the mouth leak status. For example, in some instances, the determined mouth leak status can be used to update the AHI number and/or the therapy number calculation, as otherwise mouth leak may look like an apnea (e.g. the AHI number and/or the therapy score can be higher than what is accurate). A therapy number or score can comprise, or be derived from, one or more metrics selected from therapy usage time of the sleep session; AHI for the session; average leak flow rate for the session; average mask pressure for the session; number of sub-sessions within the session; sleep status and/or sleep stage information; and whether the session is a compliant session according to a compliance rule. One example of a compliance rule for CPAP therapy is that a user, in order to be deemed compliant, is required to use the respiratory system for at least four hours a night for at least 21 of 30 consecutive days. As will be understood, other such compliance rules may be selected.

In some such implementations, for the calculating and/or modifying the AHI score and/or the therapy score, sensor data associated with the user during the sleep session is received from a sensor coupled to the respiratory device. The sensor data is indicative of a number of sleep-disordered breathing events during the sleep session. The AHI score and/or the therapy score is determined based, at least in part, on the number of sleep-disordered breathing events. The mouth leak status is correlated with the sensor data to output one or more false positive sleep-disordered breathing events. The one or more false positive sleep-disordered breathing events are subtracted from the number of sleep-disordered breathing events to output a modified number of sleep-disordered breathing events. The AHI score and/or the therapy score is calculated based, at least in part, on the modified number of sleep-disordered breathing events.

For example, in some implementations, the mouth leak status can include a duration of mouth leak and/or a severity of mouth leak. Based at least in part on the duration of mouth leak and/or the severity of mouth leak, a sleep or therapy score (e.g., the sleep or therapy score described herein) is modified (e.g., lowered or decreased). The sleep score referred to herein is exemplified by the ones described in International Publication No. WO 2015/006364, such as at paragraphs [0056]-[0058] and [0278]-[0285], which is hereby incorporated by reference herein in its entirety. Alternative definitions are also possible.

Furthermore, an over titrated (e.g., high) pressure setting can promote unwanted mouth leak. As such, in some implementations, the method 300 further includes step 332, where pressure settings of the respiratory device are adjusted based at least in part on the mouth leak status. For example, the system 100 can be configured to adjust the pressure level down and/or recommend to a qualified person and/or an intelligent system to make and/or approve this therapy change.

In some implementations, the respiratory system 120 includes an AutoSet function for the RPT. An AutoSet module enables the RPT to change pressure level throughout the night based on a user's needs. Undetected mouth leak can lead to the RPT falsely determine that an apnea has occurred. In some instances, having mouth leak can confuse the AutoSet function (especially if the user is not already at their highest available pressure). For the period of mouth breathing, AutoSet / RPT therapy engine may think that the user is having an apnea (maybe a very long apnea), until eventually a breath is detected, and it starts to increase the pressure. After some breaths after the leak, the machine may incorrectly raise the pressure (using, for example, the Autoset) to "treat" the "apnea" that is actually a mouth leak, which leads to more mouth leak as the pressure is higher. In other words, the pressure increase can worsen the mouth leak (e.g., extend a duration of the mouth leak, and/or worsen a severity of the same). In turn, the discomfort increases, and may eventually wake the user, and/or cause the mask to be taken off, and/or worsen the dry mouth or other symptoms related to mouth leak.

In some implementations, responsive to the mouth leak status, pressure settings of the respiratory device are adjusted, where the pressure settings are associated with the pressurized air supplied to the airway of the user. In some such implementations, the acoustic data associated with the user is analyzed to determine that the user is exhaling. Responsive to the determination that the user is exhaling, a pressure of the pressurized air to the airway of the user is reduced during the exhaling of the user. In some such implementations, the reducing the pressure of the pressurized air includes increasing an Expiratory Pressure Relief (EPR) level associated with the respiratory device, which is described in more detail herein for method 500 (FIG. 5).

In some implementations, the method 300 further includes step 333, where humidification settings are adjusted, responsive to the mouth leak status of the user. For example, in some implementations, if the user has some less severe mouth leak (e.g., low severity, but leading to a feeling of dry mouth in the morning), then a higher humidity will help keep the mouth and lips moisturized - up to a point. Therefore, adjusting the humidity is a way to counterbalance dryness. The more humidity from the humidifier into the conduit and/or the tube, blown into nose, the more humidity (e.g., moisture) out through mouth. Additionally or alternatively, a substance can be released into the moisture to be introduced into the pressurized air for the adjusting the humidification settings. The substance can be stored, for example, in the receptacle 180 until a portion of it is ready to be released. The substance can include, a saline solution, a decongestant, an essential oil, a scent, a medication, or any combination thereof.

The mouth leak status may be affected by various factors. In some instances, the mouth leak status is associated with the sleep position of the user. For example, mouth leak may be more severe in non-supine positions. In other words, a side sleeper may have a higher risk of mouth leak, but conversely require less pressure if they have positional apnea. In some instances, the user sleeps on a smart pillow. In some implementations, the method 300 further includes step 334, where the smart pillow is adjusted such that the smart pillow urges the user to change position of the user's head responsive to the mouth leak status. In some instances, the user sleeps on a smart mattress. In some implementations, the method 300 further includes step 335, where the smart mattress is adjusted in response to the mouth leak status, such that the smart bed or the smart mattress urges the user to change position of the user's body.

In some implementations, the user sleeps with a wearable sensor. The wearable sensor may be coupled to and/or integrated in a watch worn by the user. In some such implementations, the method 300 further includes step 336, where the wearable sensor is adjusted in response to the mouth leak status, such that the wearable sensor stimulates a neck or a jaw of the user to close the user's mouth.

In some implementations, the method 300 includes step 337, where a notification is provided to the user (and/or a physician, healthcare provider, etc.) via a display device (e.g., the display device 172 and/or the display device 128) such that the user is alerted of the mouth leak status. The notification can include a visual notification, an audio notification, a haptic notification, or any combination thereof.

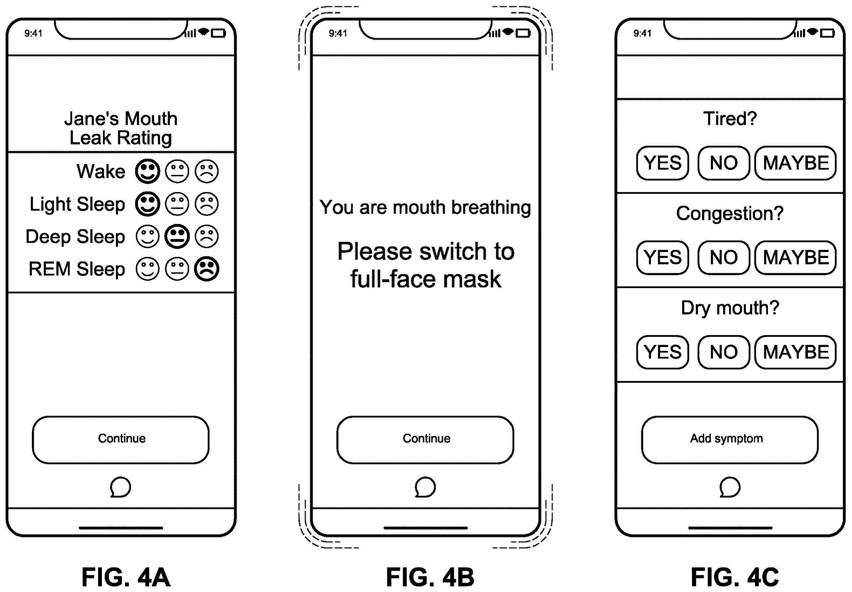

In some implementations, the notification (step 337) includes a message (visual, audio, and/or haptic) that includes a reminder for the user to (i) close his/her jaw during the sleep session (e.g., via a chin strap or similar means), (ii) moisturize lips before a next sleep session, or (iii) both (i) and (ii). Alternatively or additionally, the message includes a recommendation or instruction to the user (i) to use a different mask, (ii) to wake up, (iii) that the user is having a mouth leak, or any combination thereof. Further examples of the visual notification are shown in FIGS. 4A-4C and discussed herein.

One or more of the steps of the method 300 described herein can be repeated one or more time for additional sleep sessions (e.g., a second sleep session, a third sleep session, a fifth sleep session, a tenth sleep session etc.). As such, acoustic data may be received and accumulated over several sleep sessions. If analysis of the accumulated data suggests that the user is regularly mouth breathing during sleep sessions, the user may have the wrong type of mask (e.g., nasal mask or nasal pillows) when a full face mask would be more appropriate for their breathing.

The user would have stopped using therapy as a result of their regular mouth leak, when proactively being provided with a "better" (more suitable to them) full face mask would be a better outcome. Therefore, in some implementations, if the user is regularly mouth breathing, the method 300 provides for recommending (or automatically causing to be drop shipped to the user) a more suitable mask. Additionally or alternatively, the method 300 provides for a medically approved AI system to automatically generate a prescription for the more suitable mask (e.g., a current user of a nasal mask or a nasal pillow may receive a recommendation for a full-face mask). A full-face mask user is less likely to experience mouth leak than a nasal mask user. Therefore, a mouth-breathing user can be trained with a full-face mask, over time, to stop the habit of mouth breathing, and then go back to a nasal mask.

Other examples of subsequent actions after detection of regular mouth breathing behaviors of a user include: recommending (or automatically causing to be drop shipped to the user) a chin strap, which may help keep the jaw closed at night; and/or recommending (or automatically causing to be drop shipped to the user) a nasal cradle cushion and/or another suitable cradle, instead of the standard cushion. A different cradle can provide enhancement to the mask to provide a good seal even when the user is sleeping in different positions.

FIG. 4A illustrates a visual indicator of a mouth leak rating (e.g., a mouth leak score) for a user on a display device. The mouth leak score can be determined based, at least in part, on a percentage of time the user experiences mouth leak during the sleep session (e.g., a duration of mouth leak as a percentage of the total therapy time), a mouth leak peak volume, a mouth leak total volume, or any combination thereof. In some implementations, sleep stage data associated with the user during the sleep session is received. The sleep stage data is analyzed to determine a sleep stage. The sleep stage can include wake (wake, drowsy), sleep (non-REM light sleep N1, N2, deep sleep N3, REM sleep), sleep stage fragmentation (due to for example, residual apnea), hypopnea, or any combination thereof. The mouth leak status (which can include one or more of time, duration, and frequency of mouth leak) and/or the mouth leak score can be associated with the determined sleep stage, which thus allows mouth leak to be correlated, at least in part, with sleep stage.

As shown in FIG. 4A, a visual indication for Jane includes a separate mouth leak score per sleep stage displayed on a mobile phone. Jane's mouth leak rating shows a choice of three emoticons per sleep stage. Determining a mouth leak status for each sleep stage can be helpful to adjust the therapy customized for each sleep stage, in order to increase an overall sleep quality. For Jane, she has little to none mouth leak during the wake stage and the light sleep stage, earning her a "happy face" emoticon. She has some mouth leak during the deep sleep stage, earning her an "OK face" emoticon. She has severe mouth leak during the REM sleep stage, earning her a "sad face" emoticon. Therefore, pressure settings and/or humidification settings can be adjusted specific to the REM stage, because Jane is more likely to have a mouth leak during the REM stage.

FIG. 4B illustrates a visual indicator of a message associated with a mouth leak status of a user on a display device. The message can be any suitable message provided to the user such that the user is alerted of the mouth leak status (e.g., step 337 of the method 300). As shown, the message in FIG. 4B includes a reminder to the user to switch to a full-face mask because she is mouth breathing.

FIG. 4C illustrates a user interface displayed on a display device for receiving user feedback from a user. As shown, user input data is received from the display device (that is the same as, or similar to, the user device 170) after a sleep session. The user can provide subjective feedback regarding the sleep quality and/or symptoms experienced during the sleep session. Based at least in part on the user input data, the mouth leak score (FIG. 4A) can be modified. The user input data can also be included in one or more steps of any of the methods described herein to aid in determining the mouth leak status, including, for example, step 330 of the method 300, step 530 and/or 540 of the method 500, step 640 of method 600.

Referring to FIG. 5, a method 500 for determining an optimal inhalation pressure and an optimal exhalation pressure for a user is illustrated. One or more steps of the method 500 can be implemented using any element or aspect of the system 100 (FIGS. 1 and 2A-2B) described herein. The method 500 can also be used in conjunction with one or more steps of the method 300.

Step 510 of the method 500 includes receiving inhalation pressure data and exhalation pressure data associated with pressurized air supplied to a user during a plurality of sleep sessions. For example, in some implementations, the inhalation pressure data and the exhalation pressure data are generated via at least of the one or more sensors 130 (FIG. 1), such as the pressure sensor 132.

Step 520 of the method 500 includes receiving inhalation acoustic data and exhalation acoustic data associated with the user during the plurality of sleep sessions. For example, in some implementations, the inhalation acoustic data and the exhalation acoustic data are generated via at least of the one or more sensors 130 (FIG. 1), such as the microphone 140. Step 520 can be the same as, or similar to, step 310 of the method 300.

Step 530 of the method 500 includes analyzing the inhalation acoustic data and the exhalation acoustic data associated with the user. Step 530 can be the same as, or similar to, or duplications of, step 320 of the method 300. In some implementations, the inhalation acoustic data and the exhalation acoustic data are analyzed to determine a mouth leak status. The determination step is the same as, or similar to, or duplications of, step 330 of the method 300.

Step 540 of the method 500 includes determining an optimal inhalation pressure and an optimal exhalation pressure for the user, based at least in part on (i) the mouth leak status of the user for each sleep session of the plurality of sleep sessions and (ii) the pressure data.

In some implementations, the method 500 further includes step 550, where the optimal inhalation pressure and the optimal exhalation pressure are set as the pressure settings for the pressurized air supplied to the user for a subsequent sleep session. Alternatively, the pressure settings are slowly adjusted to avoid abrupt changes, if the current pressure settings are much different from the optimal pressures.

The method 500 can also include a feedback loop to evaluate whether the adjustment has had the desired outcome, and/or whether the pressure level needs to be increased or decreased. For example, subsequent acoustic data during a subsequent sleep session is received from the microphone. The optimal inhalation pressure and the optimal exhalation pressure are received as subsequent pressure data for the subsequent sleep session. The analyzing step (530) and the determining step (540) are repeated to update the optimal inhalation pressure and the optimal exhalation pressure for the user (550).

Additionally or alternatively, the method 500 can include a machine learning algorithm (similar to the machine learning algorithm in method 600) that determines whether the user is having a real apnea or just a mouth leak "disguised" as an apnea. Based on the determination, the pressure level is either further increased (e.g., to treat the real apnea that current pressure level is not managing to treat) or kept the same (or even reduced).

For example, in some implementations, the respiratory device may include an Expiratory Pressure Relief (EPR) module. The EPR module can have different settings for an EPR level, which is associated with the difference between a pressure level during inspiration and a reduced pressure level during expiration. Activating and/or adjusting an EPR level (e.g., setting a relatively lower expiration pressure) may reduce mouth leak, based on the determined optimal inhalation pressure and the optimal exhalation pressure (step 550). The EPR level may also be adjusted during specific sleep stages, as discussed herein.

Referring to FIG. 6, a method 600 for estimating a mouth leak status for a user using a machine learning algorithm is illustrated. One or more steps of the method 600 can be implemented using any element or aspect of the system 100 (FIGS. 1 and 2A-2B) described herein. The method 600 can also be used in conjunction with one or more steps of the method 300 and/or one or more steps of the method 500.

Even when a user has a "good" (e.g., properly fitted) mask for them in the more general sense, they may be congested and/or ill one day such as during one or more sleep sessions. The user may temporarily need different settings and/or intervention in order to minimize the risk of mouth leak during a sleep session when congested and/or ill. Therefore, in some implementations, the method 600 allows for predicting if a user is likely to have mouth leak in one or more sleep sessions, and take action and/or recommend action to reduce or mitigate this risk. For example, for some people, alcohol consumption may lead to more mouth leak due to the relaxant effect; and dehydration caused by alcohol may also affect lip seal. The common cold or influenza may lead to more mouth leak, due to congestion.

Step 610 of the method 660 receiving acoustic data associated with a user of a respiratory device during a plurality of sleep sessions. For example, in some implementations, the acoustic data is generated via at least of the one or more sensors 130 (FIG. 1), such as the microphone 140. Step 610 can be the same as, or similar to, step 310 of the method 300 and/or step 520 of the method 500.

Step 620 of the method 660 includes receiving physiological data associated with the user for the plurality of sleep sessions. For example, in some implementations, the physiological data is generated via at least of the one or more sensors 130 (FIG. 1). The physiological data can be generated as described herein, for example, with reference to the method 300. Some examples of the physiological data generated by the sensor are: breath alcohol data, blood alcohol data, blood pressure data, blood glucose data, congestion data, occlusion data, body temperature data, heart rate data, movement data, respiration data (e.g., a respiration rate and/or a respiration shape), sleep stage data, mask data, and $CO_2$ level data.

Step 630 of the method 660 includes analyzing the acoustic data to determine a mouth leak status of the user for each sleep session of the plurality of sleep sessions. Step 530 can be the same as, or similar to, or duplications of, step 320 and/or step 330 of the method 300.

Step 640 of the method 660 includes training a machine learning algorithm with (i) the mouth leak status of the user for each sleep session of the plurality of sleep sessions and (ii) the physiological data, such that the machine learning algorithm is configured to receive as an input current physiological data associated with a current sleep session, and determine as an output an estimated mouth leak status for the current sleep session. The training of the machine learning algorithm may include analyzing acoustic data and/or airflow data corresponding to known mouth leak events (identified by, for example, a camera).

One of more of the steps 610 to 640 of the method 600 described herein can be repeated to create a feedback loop similar to what is described with reference to the method 500. The feedback loop allows continuous improvement of the machine learning algorithm to adapt with the user.

The machine learning algorithm can be used in various implementations. For example, in some implementations, the current physiological data during the current sleep session is received as the input to the machine learning algorithm (step 650). The estimated mouth leak status for the current sleep session is generated as the output of the machine learning algorithm (step 652). Based at least in part on the estimated mouth leak status, pressure settings of the respiratory device are adjusted (step 654).

For further example, in some implementations, the current physiological data prior to the next sleep session is receive as the input to the machine learning algorithm (step 660). The estimated mouth leak status for the next sleep session is generated as the output of the machine learning algorithm (step 662). Based at least in part on the estimated mouth leak status, a recommended adjustment is determined for displaying on a user device (step 664).

Some examples of the recommended adjustment include: (i) adjusting pressure settings of the respiratory device, the pressure settings being associated with the pressurized air supplied to the airway of the user; (ii) adjusting humidification settings of a humidifier coupled to the respiratory device, the humidifier being configured to introduce moisture to the pressurized air supplied to the airway of the user; (iii) recommending a mask type for the respiratory device, (iv) recommending a sleep position for the user, (v) recommending a chin strap for the user; and (vi) recommending a nasal cradle cushion for the user. The recommended adjustment can be displayed in a similar manner as in FIG. 4B and its corresponding description, and/or in step 337 of the method 300.

Furthermore, in some implementations, data generated by the method 600 can provide for classification of physiological factors related to mouth leak that may cause irritation (e.g., causing mask removal, causing disruption to sleep stages, causing changes to heart rate, causing reported symptoms next morning such as dry mouth).

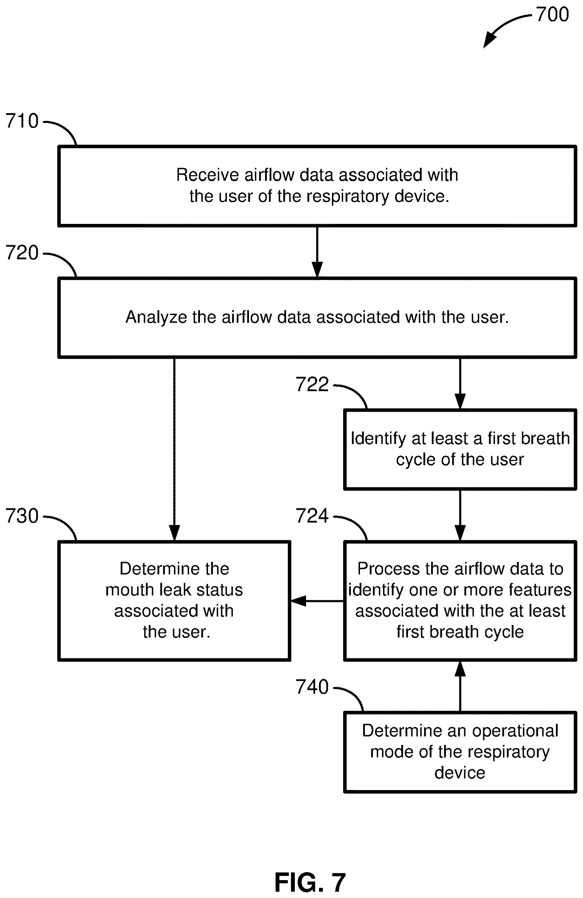

Referring to FIG. 7, a method 700 for determining a mouth leak status associated with a user of a respiratory device is disclosed, according to some implementations of the present disclosure. At step 710, airflow data associated with the user of the respiratory device (e.g., the respiratory device 122 of the system 100 shown in FIG. 1) is received. At step 720, the airflow data associated with the user is analyzed. In some implementations, the analyzing the airflow data associated with the user includes processing the airflow data to identify one or more features that distinguish mouth leak from (i) normal respiration during therapy and/or (ii) other types of unintentional leak (e.g., unintentional leak from the user interface). Based at least in part on the analysis, at step 730, the mouth leak status (e.g., no mouth leak, valve-like mouth leak, continuous mouth leak) associated with the user is determined. In some implementations, the mouth leak status is indicative of whether or not air is leaking from the mouth of the user.

The airflow data can include pressure data, which is associated with the pressure signal within the respiratory system, such as mask pressure measured by the respiratory system. In some implementations, the airflow data further includes flow rate data. In some such implementations, the airflow data may be received from a flow rate sensor (e.g., the flow rate sensor 134 of the system 100) associated with the respiratory device; the pressure data may be received from a pressure sensor (e.g., the pressure sensor 132 of the system 100) associated with the respiratory device.

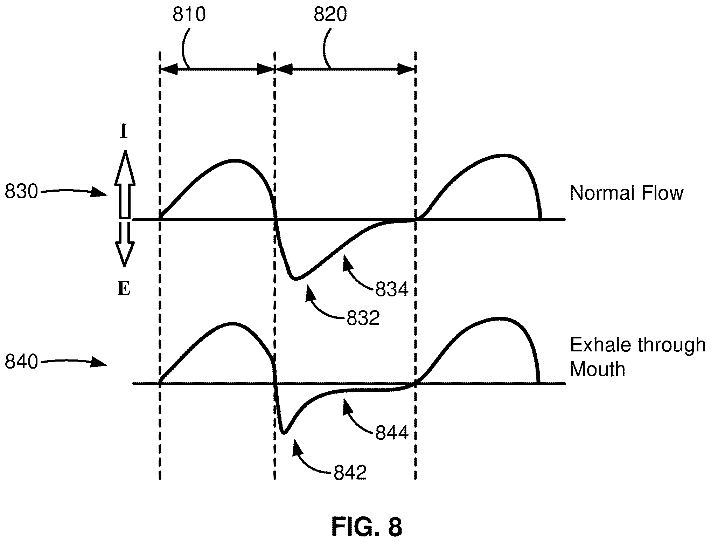

In some implementations, within the received airflow data (step 710) and/or using the analyzed airflow data (step 720), at least a first breath cycle of the user is identified at step 722. For example, in some such implementations, two breath cycles, three breath cycles, four breath cycles, five breath cycles, six breath cycles, seven breath cycles, or eight breath cycles can be identified at step 722 and later processed at step 724. The first breath cycle can include an inhalation portion (e.g., inhalation portion 810 in FIG. 8) and an exhalation portion (e.g., exhalation portion 820 in FIG. 8). The first breath cycle (and/or additional breath cycles) may be determined by any suitable methods, such as disclosed herein. In some examples, the first breath cycle can be determined by using an average length of breath for the user, such as about five seconds. In some examples, the first breath cycle can be identified based at least in part on the received airflow data from step 710. In some examples, the identifying the at least first breath cycle (step 722) includes identifying a beginning of the first breath and/or an end of the first breath. The beginning and/or the end of the first breath signifies the transition between the first breath and its adjacent breath.

In some implementations, at step 724, the airflow data is processed to identify one or more features associated with at least the first breath cycle. For example, in some such implementations, the airflow data is processed to identify one or more features associated two breath cycles, three breath cycles, four breath cycles, five breath cycles, six breath cycles, seven breath cycles, or eight breath cycles. The one or more features can include a pressure range, a minimum pressure, a maximum pressure, a pressure skewness, a pressure kurtosis, a pressure power spectral density (e.g., the pressure power spectral density in the range of 1-3 Hz), a flow rate range, a minimum flow rate, a maximum flow rate, a flow skewness, a flow kurtosis, a flow sub-area ratio (e.g., a ratio of the expiratory peak area over total expiratory area of the flow rate data), or any combination thereof. In some implementations, specific combinations of the one or more features are used to determine the mouth leak status, such as the combination of the pressure range, minimum pressure, and the flow sub-area ratio. Each of the one or more features may be determined and/or extracted from detrended pressure data and/or detrended flow rate data (as discussed in more detail below). In some such implementations, the pressure range and the minimum pressure are determined and/or extracted from the detrended pressure data; and the flow sub-area ratio is determined and/or extracted from the detrended flow rate data.

Additionally or alternatively, in some implementations, the one or more features include spectral features based on the pressure data. For example, as valve-like mouth leak tends to manifest as sharp variations in pressure, the pressure signal exhibits and/or plots as a high peak in the Power Spectral Density of the pressure signal at high frequencies. A FFT can be taken on windows of five seconds of the pressure signal, and the peak value at high frequencies (e.g., 1-3 Hz) is computed for each window. Additionally or alternatively, in some implementations, the one or more features include skewness and/or kurtosis of the, optionally detrended, pressure signal, which can also characterize sharp variations and/or asymmetry in the pressure signal. Further, in some implementations, the same computations applied on the pressure data can also be applied on the airflow data to extract additional features to be used to determine the mouth leak status.

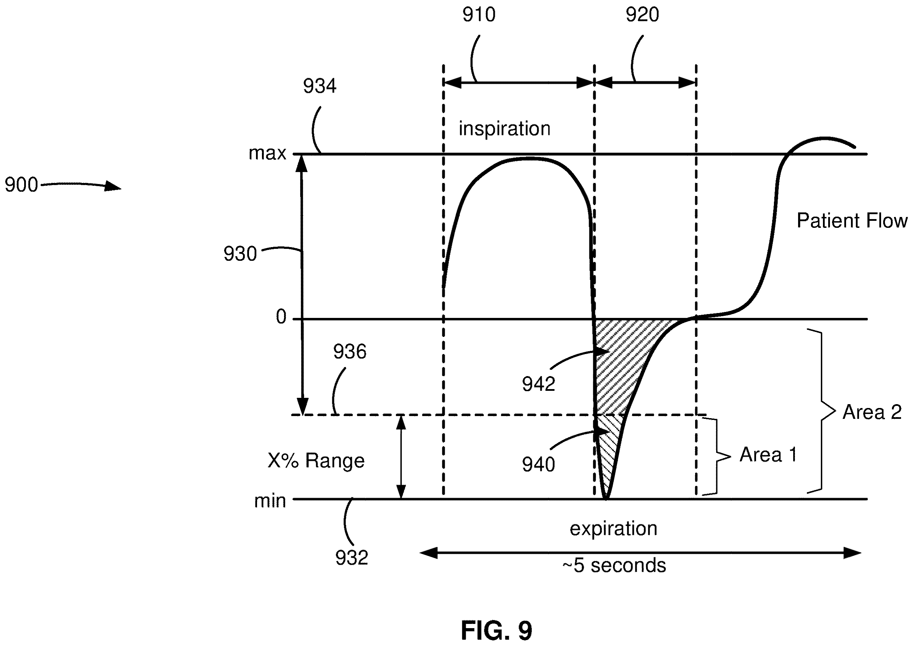

Some of those features are discussed in more detail with reference to FIG. 9. In some examples, the one or more features associated with at least the first breath cycle are calculated over 1, 2, 3, 4, 5, 6, 7, or 8 adjacent, such as consecutive, breath cycles. In some examples, the one or more features associated with the first breath cycle are calculated over a predetermined duration of time, e.g., 30 seconds. That is because in some cases, mouth leak tends to occur in trains of breaths. Therefore, statistics over multiple breaths can be analyzed to rule out "one-off" events that can result in the alteration of just one isolated breath, and/or events that are in fact associated with other processes (e.g. the user gasping, an apnea, or the like).

In some implementations, before extracting any features based on the pressure data, the pressure data (e.g., pressure time trace) is detrended to account for the effect of Expiratory Pressure Relief (EPR) or AutoSet. EPR effectively ramps up pressure during inhalation, and drops the pressure down at the beginning of exhalation (holding the value low during the entire exhalation phase). AutoSet increases the therapy pressure after the onset of a respiratory event, and decreases the therapy pressure once the user no longer exhibits the respiratory event. These pressure variations are independent of mouth leak, and can result in a change in the minimum pressure, the maximum pressure, and the pressure range. Therefore, under certain operational modes, these pressure variations need to be accounted for, resulting in the detrended pressure data, such as the detrended minimum pressure. Once the trend is removed from the pressure time series, the detrended minimum pressure, maximum pressure, and/or pressure range may be extracted to be analyzed for the mouth leak status under those operational modes. Additionally or alternatively, the features derived from the flow rate signal can be detrended in the same, or similar fashion.

For example, in some implementations, at step 740, an operational mode (e.g., CPAP, APAP, or BiPAP) of the respiratory device is determined. In some such implementations, the one or more features are determined (step 724) based at least in part on the determined operational mode (step 740). For example, the one or more features may be determined (step 724) based at least in part on removing an Expiratory Pressure Relief (EPR) component in the pressure data (received at step 710).

In some implementations, the one or more features may then be fed into a logistic regression model to determine the mouth leak status (step 730). For example, these features can be inputted in the logistic regression model, which outputs a probability (e.g., a single number). A threshold is then applied on this probability to determine the mouth leak status (e.g., whether the user is experiencing any mouth leak). In some examples, the threshold for the probability indicative of mouth leak is 0.6.

In some examples, for any given epoch (e.g., a 30-second range, or on a breath-by-breath basis), the threshold can be calculated using the following formula:

$$p = \frac{1}{1 + e^{-(b + \alpha_1 x_1 + \alpha_2 x_2 + \alpha_3 x_3)}}$$

where $x_1$ is the pressure range, $x_2$ is the detrended minimum pressure, and $x_3$ is the flow sub-area ratio for the given epoch. $\alpha_1$, $\alpha_2$, $\alpha_3$ are the weights of the logistic regression. b is the bias. In this example, the values for $\alpha_1$, $\alpha_2$, $\alpha_3$ are -6.12339829, 0.87103483, -5.26285759, respectively; and the value for b is -1.2533223418287587. If p > 0.6, the epoch is classified as containing mouth leak, otherwise the epoch is marked as negative (e.g., no mouth leak).

Although this example relates to the three features (i.e., the pressure range, the minimum pressure, and the flow sub-area ratio), other features, and more or fewer features may be used. In some implementations, the number of weights and/or their values in the formula will change based at least in part on the features considered and/or the training data available. Additionally or alternatively, in some implementations, the probability threshold p can be a dynamic value modified over time, modified based on a desired sensitivity and/or specificity in the system, or modified based on a particular user; and thus the probability threshold p can be a tunable value. For example, the probability threshold p can be > 0.25, 0.3, 0.35, 0.4, 0.45, 0.5, 0.55, 0.6, 0.65, or 0.7 for the epoch to be classified as containing mouth leak.

Referring briefly to FIG. 8, flow rate versus time plots showing a first breath 830 and a second breath 840 are illustrated, according to some implementations of the present disclosure. As will be understood, "I" is the inhalation portion, and "E" the exhalation portion, of the first breath 830. The first breath 830 corresponds to a user breathing normally. The second breath 840 corresponds to the user exhaling through their mouth (i.e. mouth leak). As shown, when the user is exhaling through their mouth, the beginning of the exhalation portion 820 has a sharper peak 842 compared to the corresponding peak 832 when the user is breathing normally. This "sharpness" of the peaks can be measured using the method 700 (e.g., as one of the features being processed in step 724) and/or illustrated in FIG. 9. For example, the "sharpness" of the peaks can be determined using the flow sub-area ratio described herein.

Additionally or alternatively, in some implementations, when the user is exhaling through their mouth, after the peak 842, the exhalation portion 820 has a flatter curve 844 compared to the corresponding curve 834 when the user is breathing normally. In some such implementations, this degree of expiratory flattening after the peaks can be measured using the method 700 (e.g., as one of the features being processed in step 724) and/or illustrated in FIG. 9. For example, the degree of expiratory flattening can be determined by (i) calculating the skewness and/or kurtosis of the flow signal, and/or (ii) assessing the length of the interval on which the derivative of the flow signal is close to zero and/or the standard deviation of the flow signal is close to zero.

Referring now to FIG. 9, a plurality of features identified within a breath cycle 900 is illustrated, according to some implementations of the present disclosure. The breath cycle 900 includes an inhalation portion 910, and an exhalation portion 920. The inhalation portion 910 and/or the exhalation portion 920 may be determined using one or more steps of the method 700, such as step 720 and/or step 722. The plurality of features may be identified using one or more steps of the method 700, such as step 724.

In some implementations, the plurality of features can include features based on the flow rate, and features based on pressure. For example, the features based on the flow rate can include minimum flow rate, maximum flow rate, flow rate range, ratio of the expiratory peak over total exhalation area (or "flow sub-area ratio"), skewness of the flow, kurtosis of the flow, degree of the flattening on expiration, or any combination thereof. The features based on pressure can include minimum pressure, maximum pressure, pressure range, power spectral density of the pressure in the range 1-3 Hz, skewness of the pressure signal, kurtosis of the pressure signal, or any combination thereof. In some such implementations, the features based on the flow rate and/or the features based on the pressure can be derived after a detrending operation on the flow rate signal and/or the pressure signal was applied.

Still referring to FIG. 9, a flow rate range 930, a minimum flow rate 932, and a maximum flow rate 934 are shown. The minimum flow rate 932 and the maximum flow rate 934 can be used as intermediary steps for deriving the ratio of the expiratory peak over total exhalation area. In some such implementations, the minimum flow rate 932 is associated with an end of the inhalation portion 910 and/or a beginning of the exhalation portion 920. In some implementations, boundaries of the flow rate range 930 are defined by the minimum flow rate 932 and the maximum flow rate 934.

In some implementations, the plurality of features further includes the flow sub-area ratio, which can be calculated by dividing a first sub-area 940 from a second sub-area 942. The first sub-area 940 is defined by an area calculated from the minimum flow rate 932 to a flow threshold level 936. In some implementations, the flow threshold level (e.g., a cut-off level, which can be the delineation level for the expiratory peak) is set as an intermediary step to derive the ratio of expiratory peak over total expiration area (or "flow sub-area ratio"): first the minimum flow rate 932 and the maximum flow rate 934 are determined, then the flow threshold level is determined as a set percentage of the range. In some such implementations, 25% of the distance between the minimum flow rate 932 and the maximum flow rate 934 is selected to be the flow threshold level 936. Additionally or alternatively, the flow threshold level 936 is tunable.

To calculate the flow sub-area ratio, the first sub-area 940 (e.g., Area 1) is the area under the flow threshold level 936 (shown in FIG. 9 as the horizontal dashed line). In some implementations, the first sub-area 940 characterizes the sharpness of the expiration peak. The second sub-area 942 is defined by an area calculated from the minimum flow rate 932 to zero (i.e. the flow rate at the point between inspiration and expiration, or between expiration and inspiration). For example, the second sub-area 942 (Area 2) is the area under the zero line, and includes all exhalation area. The flow sub-area ratio is then calculated by dividing the first sub-area 940 by the second sub-area 942 (e.g., Area 1 / Area 2). In some such implementations, the flow threshold level 936 can be a dynamic value modified over time, modified based on a desired sensitivity and/or specificity in detection of mouth leak, or modified based on a particular user; and thus the flow threshold level 936 can be a tunable value. For example, in some implementations, the flow threshold level 936 is adjusted based at least in part on further analyzing the airflow data associated with the user (step 720 of the method 700 as shown in FIG. 7).

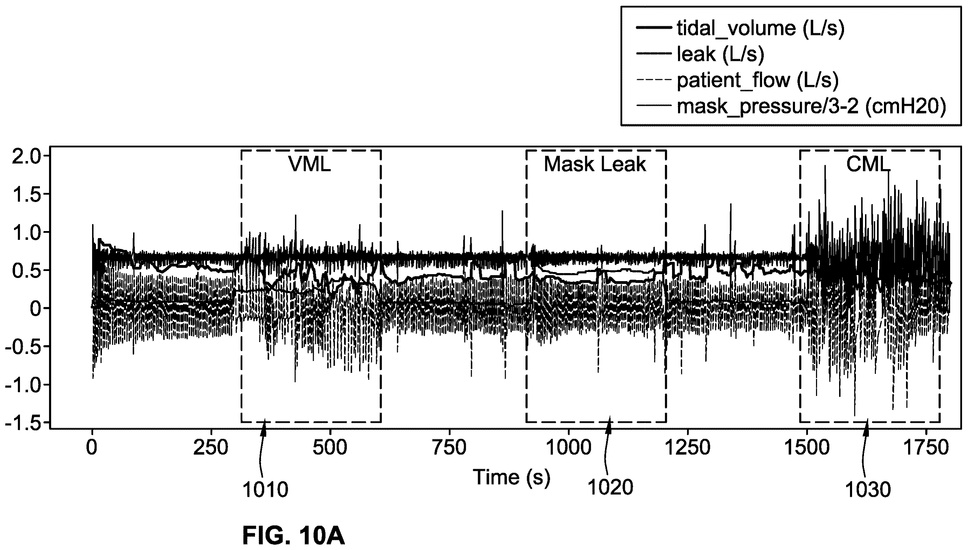

In some implementations, to differentiate between the valve-like mouth leak and continuous mouth leak, the flow rate range 930 is analyzed. In some implementations, valve-like mouth leak can be characterized by a small value of the flow sub-area ratio feature. Conversely, larger value can correspond to no mouth leak (and/or continuous mouth leak). Thus, in some such implementations, when a user is experiencing continuous mouth leak, the flow rate range 930 becomes greater than that of the user experiencing valve-like mouth leak or no mouth leak. This difference is illustrated herein in FIGS. 10A-10D, for example.

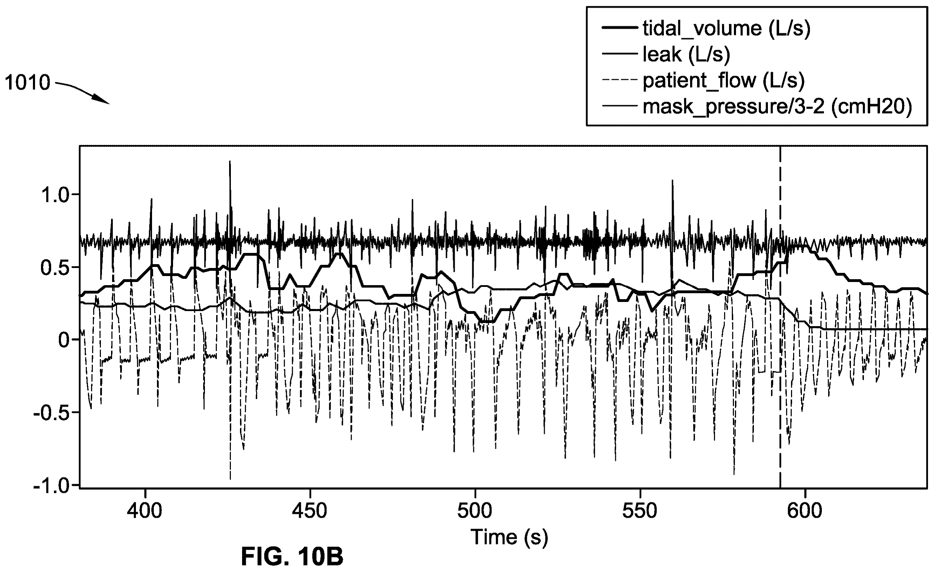

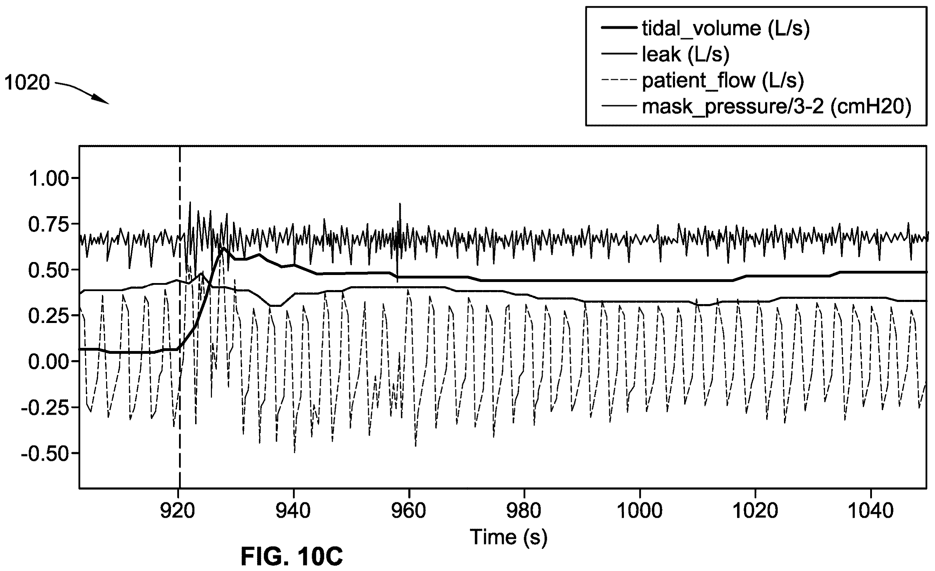

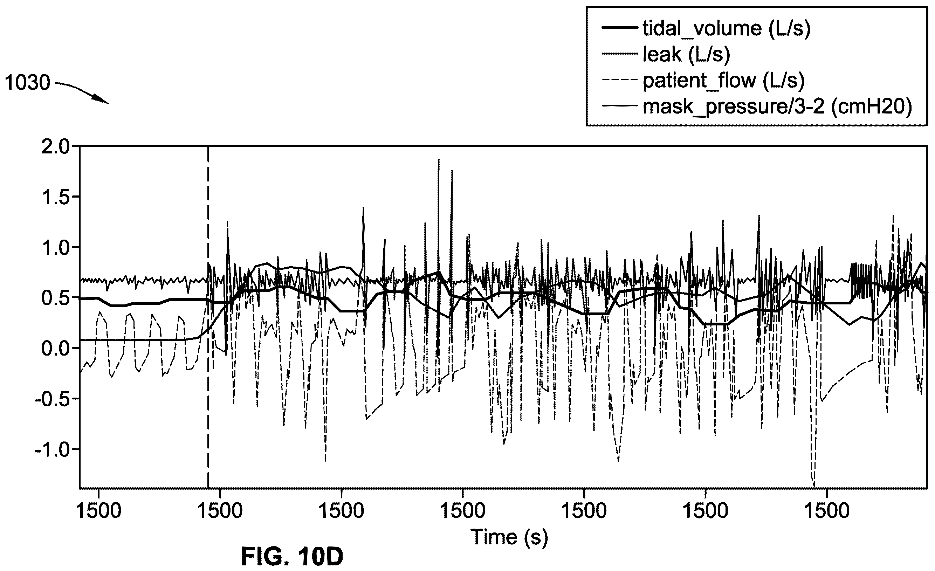

Referring generally to FIGS. 10A-10D, FIG. 10A illustrates lab data measured during a therapy session of a user displaying valve-like mouth leak (therapy session 1010), mask leak (therapy session 1020), and continuous mouth leak (therapy session 1030). FIG. 10B illustrates the therapy session 1010 of the lab data of FIG. 10 of the user displaying the valve-like mouth leak, with the dashed line indicating the end of the valve-like mouth leak event. FIG. 10C illustrates the therapy session 1020 of the lab data of FIG. 10 of the user displaying the mask leak, with the dashed line indicating the onset of the mask leak event. FIG. 10D illustrates the therapy session 1030 of the lab data of FIG. 10 of the user displaying the continuous mouth leak, with the dashed line indicating the onset of the continuous mouth leak event. As shown, the patient flow, mask pressure, tidal volume, and calculated leak are illustrated. In some implementations, the pressurized air supplied to the airway of the user during the therapy session is between 4 cmH$_2$O to 20 cmH$_2$O. In this example as shown in FIGS. 10A-10D, the pressurized air supplied to the airway of the user during the therapy session is about 8 cmH$_2$O.

Referring specifically to FIG. 10A, the mask pressure varies greater in valve-like mouth leak (session 1010) than that in mask leak (session 1020), while varying the most in continuous mouth leak (session 1030).

Unintentional leak can include genuine mask leak (e.g., the mask seal is poor) and/or mouth leak (e.g., occurs for nasal/pillows masks). In some examples, genuine mask leak is a critical confounding factor. One of the objectives of the mouth leak detection algorithm of the present disclosure is to separate the two types of unintentional leak.

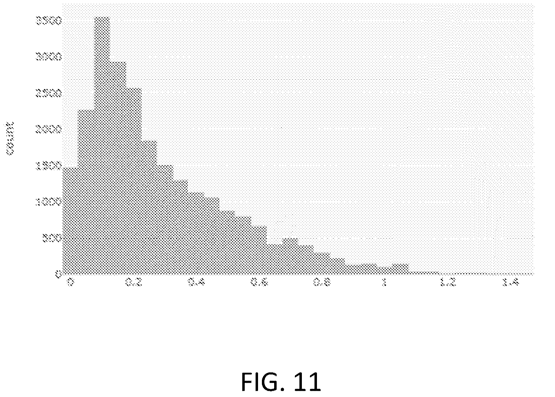

Referring to FIG. 11, a histogram of epochs with mouth leak is shown in terms of unintentional leak levels. The histogram includes data from 6 users ("Achill ECS" data), for the epochs where mouth leak was detected using a microphone attached to the mask. As shown, most epochs with mouth leak have some level of unintentional leak detected by the system (e.g., a flow generator of a respiratory therapy system).

Interim features were developed based on 143 nights from 19 users. "Achill ECS" data includes data from 6 users (with various levels of mouth leak) for 14 nights each. "Pacific ECS AUS" data includes data from 12 users (with full face mask) for 7 nights each. The "Achill ECS" data was used as clinical data to develop initial features. The "Pacific ECS AUS" data was used to test the specific features.

Features capturing slow variability (e.g., in the order of minutes) of ventilation, leak, and/or their correlation are geared towards detecting continuous mouth leak ("CML"). Features capturing fast variability (e.g., over a breath's duration) based on breath morphology are geared towards detecting valve-like mouth leak ("VML"), because faster time scales can be indicative of VML, which only happens (or to a greater extent) on expiration. In this example, a set of features that show some ability to separate the mouth leak patients was selected.

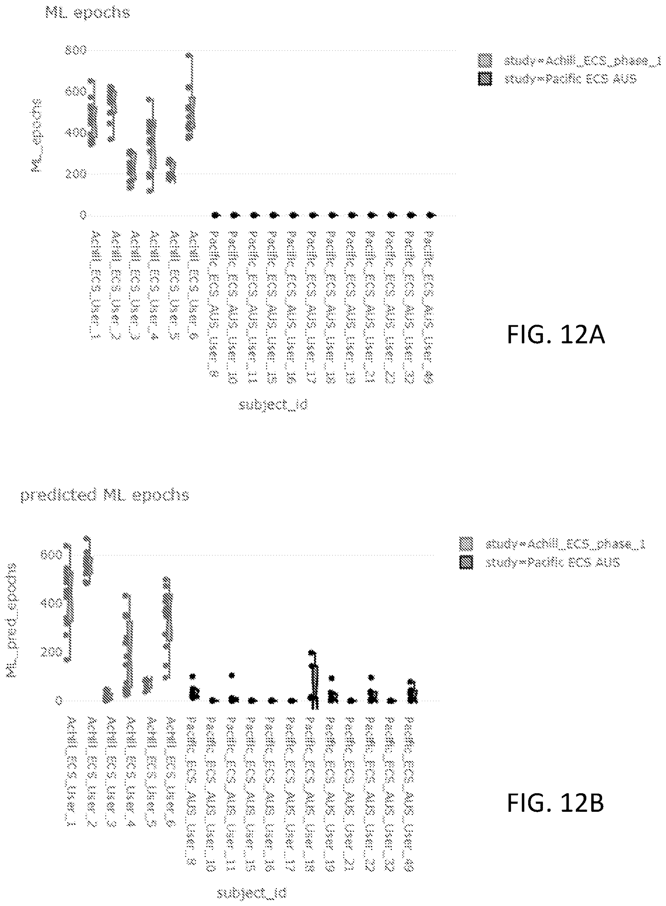

FIG. 12A illustrates the actual mouth leak duration using the "Achill ECS" data and the "Pacific ECS AUS" data. The X-axis indicates each user. The Y-axis indicates the number of epochs (in this example, 30 seconds each) measured overnight per user. As shown, because the 12 users of the "Pacific ECS AUS" data had full face masks, no actual mouth leak was detected.

FIG. 12B illustrates the predicted mouth leak duration using the "Achill ECS" data and the "Pacific ECS AUS" data. The X-axis indicates each user. The Y-axis indicates the number of epochs (in this example, 30 seconds each) measured overnight per user. The algorithm predicted the epochs, using selected features by comparing to a threshold value for each feature. As shown, the features provide good estimate of mouth leak compared to the actual mouth leak (FIG. 12A).

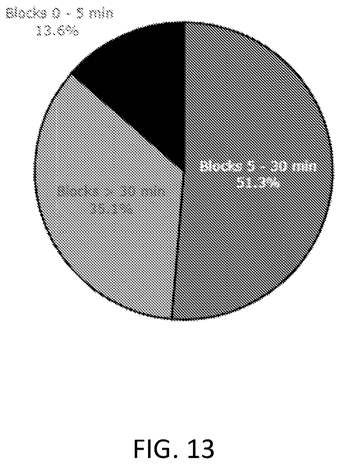

FIG. 13 illustrates proportions of scored mouth leak in terms of block duration. As shown, mouth leak is not always intermittent. Instead, mouth leak occurs typically in blocks exceeding 1 minute. Only 13.6% of scored mouth leak occurs in blocks smaller than 5 minutes, with over 30% of mouth leak occurring in blocks longer than 0.5 hour. Thus, in some implementations, such as in this example, a 30-second resolution for mouth leak features is sufficient.

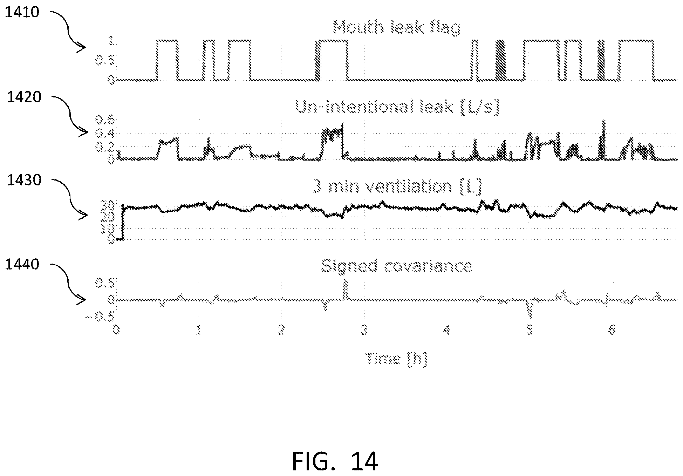

FIG. 14 illustrates signed covariance between unintentional leak and ventilation used to determine a mouth leak. In this example, the features used to estimate and/or determine the mouth leak status can include signed covariance (1440) between unintentional leak (1420) and ventilation (1430), which is used to isolate onset and offset of mouth leak events (1410). The 3-minute ventilation equals half of the integral of the absolute value of patient flow over a 3-minute window.

The onset of a mouth leak block is detected by the feature (1440) going under a set threshold (shown as "0" on FIG. 14); and the offset of the mouth leak block is detected by the feature (1440) exceeding the set threshold. In some implementations, the features used to estimate and/or determine the mouth leak status can include the time the covariance is under the set threshold (for onset), and above the set threshold (for offset). For example, the time the signed covariance holds above a threshold can be a feature.

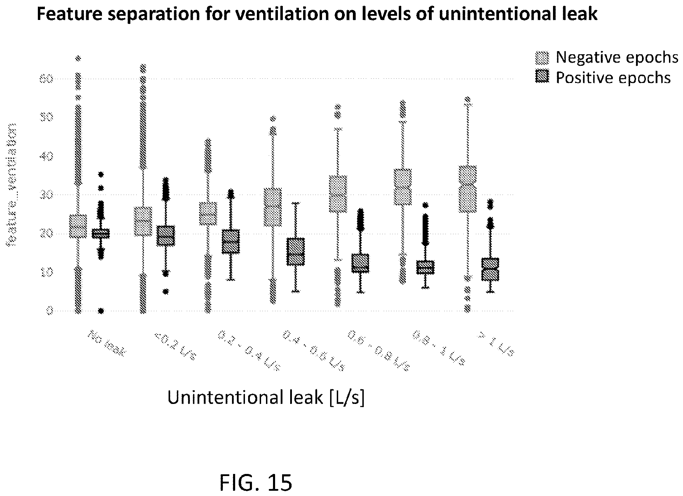

FIG. 15 illustrates the feature separation for ventilation on levels of unintentional leak. As shown, actual level of ventilation on mouth leak block has a good discriminative power by itself. While ventilation can be used as a feature directly, there can exist user bias, which may reduce the accuracy of estimating and/or determining the mouth leak status.

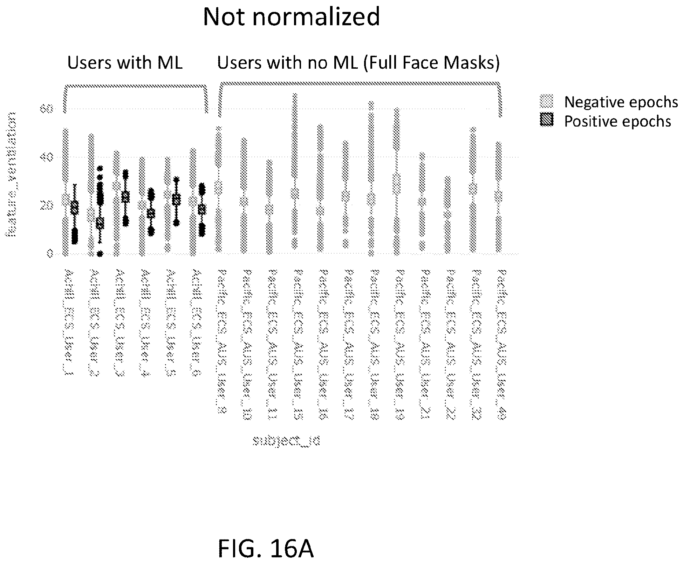

FIG. 16A illustrates negative epochs (e.g., negative for mouth leak) and positive epochs (e.g., positive for mouth leak) for each user before normalization. FIG. 16A shows clear user trends in ventilation levels (e.g., due to varied BMI and/or lung capacity among the users). In some implementations, when there is user bias, there is a baseline value that is user specific. Thus, the algorithm can be configured to (i) select periods in the record with no unintentional leak, compute average ventilation, and use it as baseline; (ii) use multiple iterations; and/or (iii) normalize after the therapy session is complete.

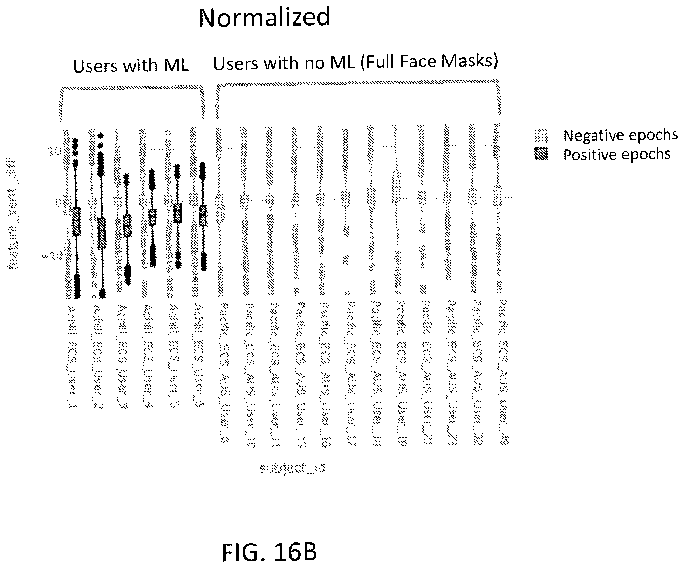

FIG. 16B illustrates negative epochs and positive epochs for each user after normalization. As shown, normalization with a baseline level increases separation. The baseline can be derived by (i) running session mean on sections with no unintentional leak, (ii) ventilation before onset of unintentional leak increasing, (iii) overall session baseline on sections with no unintentional leak, and/or (iv) user-specific baseline (e.g., from multiple nights). The normalization can be done by: (i) ratio (e.g., percent decrease with respect to baseline), and/or (ii) difference (e.g., actual decrease with respect to baseline).

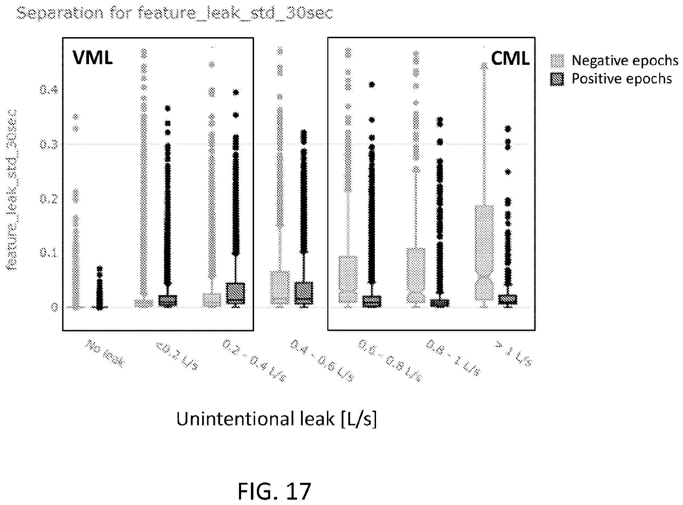

FIG. 17 illustrates the separation for the feature of unintentional leak variability. The unintentional leak variability feature is derived by taking the standard deviation of unintentional leak over a set interval (e.g. 30 seconds). In this example, high levels of unintentional leak (e.g., > 0.5 L/s) are likely associated with CML, where mouth leak is more stable than mask leak. Moderate levels of leak (e.g., <0.5 L/s) are likely associated with VML, where mouth leak is less stable than mask leak. In some implementations, the level of unintentional leak can be used for fusing more efficiently the VML and CML feature. For example, for low levels of leak, the VML features are weighted more than the CML features; for high levels of leak, the VML features are weighted less than the CML features.

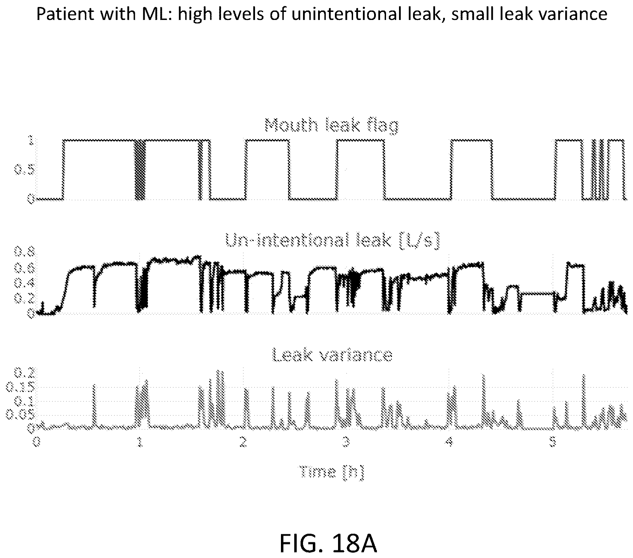

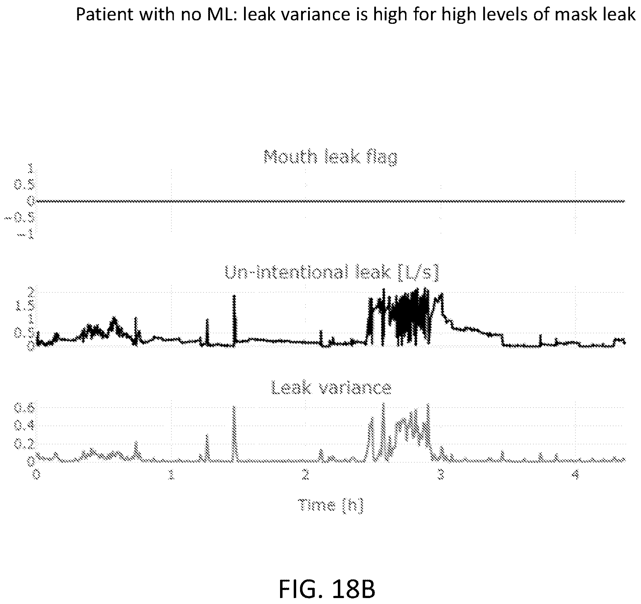

FIG. 18A illustrates an example unintentional leak variance for high levels of unintentional leak in a user with mouth leak. FIG. 18B illustrates an example unintentional leak variance for high levels of unintentional leak in a user without mouth leak. As shown in FIG. 18A, for the user with mouth leak, even though the unintentional leak level is high, the unintentional leak variance is small. In contrast, as shown in FIG. 18B, the unintentional leak variance is large for high levels of mask leak (because there is no mouth leak).

In some implementations, the features for estimating and/or determining the mouth leak status can include normalized respiration rate (e.g., similar to normalizing the ventilation), and/or the respiration rate variability (e.g., similar to the unintentional leak variability).

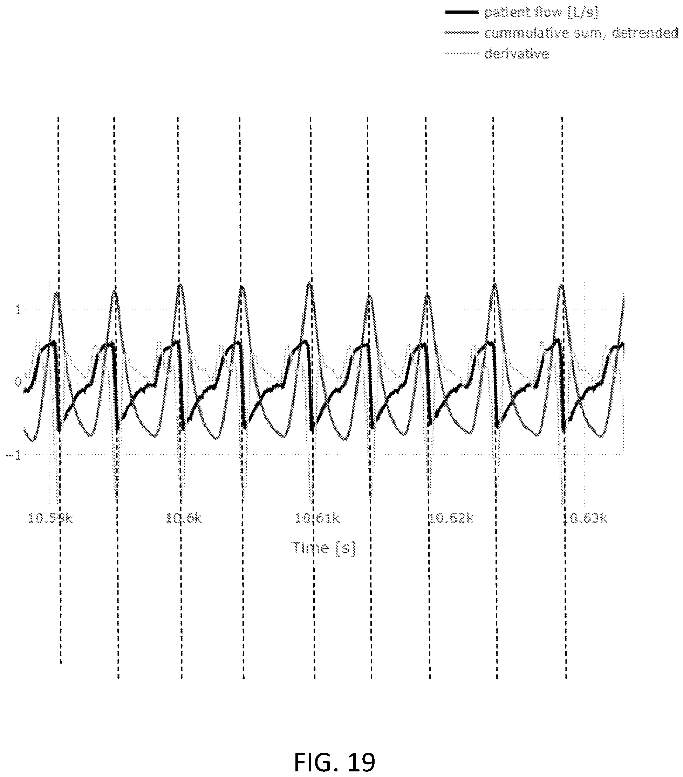

FIG. 19 illustrates breath segmentation based on flow rate data. The flow rate of a user is plotted. The derivative of the flow rate is plotted on a low-pass filter (for smoothing). The detrended cumulative sum is plotted on a high-pass filter (to better separate breath-by-breath). Each breath is segmented by taking the minima or the maxima of the plots. For example, the negative peaks of the first derivative of flow rate are used for segmentation. The positive peaks of the detrended cumulative sum are used for segmentation.

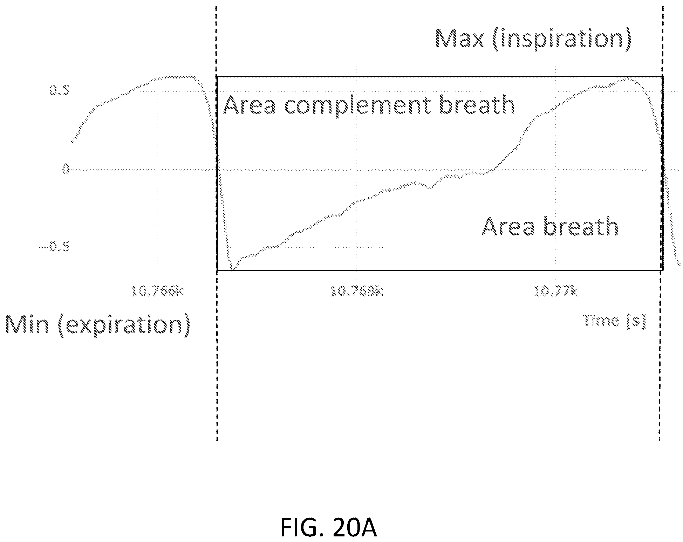

Once segmentation is done, the features can be computed on any respiratory device signal (e.g., any 25-Hz signal, such as patient flow, mask pressure, blower flow, blower pressure). Each signal can be analyzed below, totaling at least 44 features (e.g., 11+ features for each of the four signals). For example, each signal can be analyzed to compute (i) the frame area (e.g., range X duration); (ii) the breath area (AUC); (iii) the complement to the breath area; (iv) the ratio of breath area / frame area; (v) the ratio of breath area / complement to breath area; (vi) the skewness of the raw signal; (vii) the kurtosis of the raw signal; (viii) the first derivative of the skewness; (ix) the first derivative of the kurtosis; (x) the second derivative of the skewness; (xi) the second derivative of the kurtosis. For example, FIG. 20A illustrates some of these features calculated over a breath.

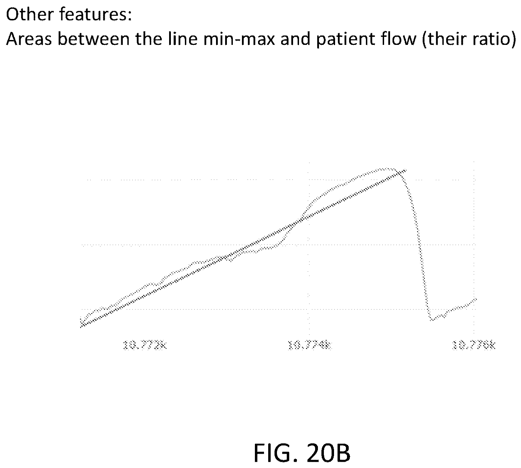

Additionally or alternatively, each signal can be analyzed for other features, such as areas between a straight line (from the minimum to the maximum) and the actual signal. For example, FIG. 20B illustrates additional breath specific features calculated over a portion of the breath. The ratio of areas above the line and under the line can be indicative of the skewness of the signal.

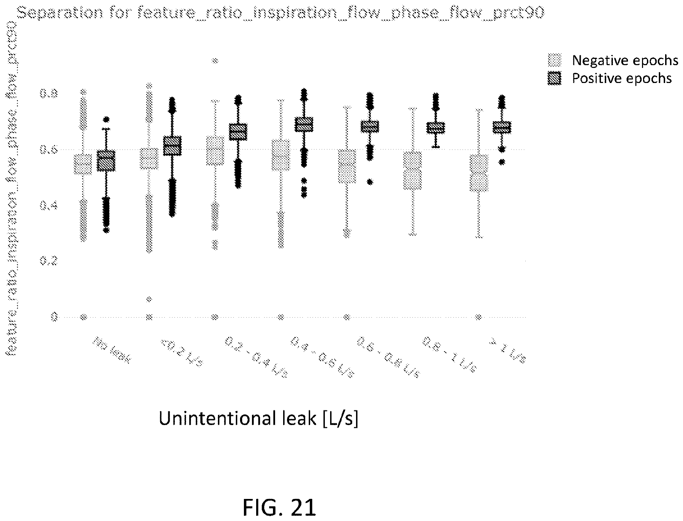

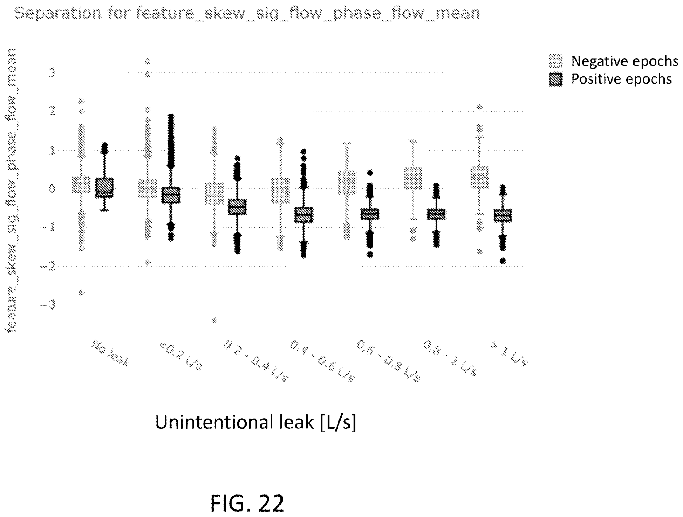

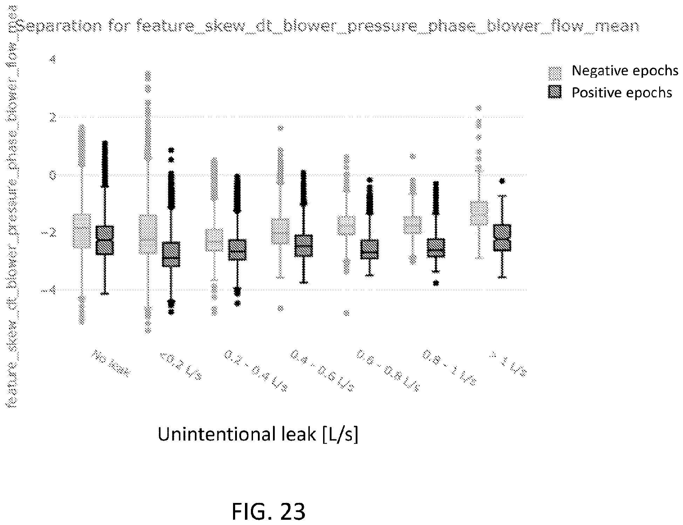

In some implementations, all breaths over a time period can be grouped in epochs (e.g. 30 seconds per epoch). Epoch based features are derived by taking statistics such as mean, median, percentiles. In some such implementations, the features can be further normalized with a baseline value, similar to the normalization described above with regard to ventilation. FIGS. 21-23 demonstrate the separability using some of the epoch based features. FIG. 21 illustrates the ratio of breath area / frame area taken on flow rate data, with epoch $90^{th}$ percentile. FIG. 22 illustrates the skewness taken on taken on flow rate data, with epoch mean. FIG. 23 illustrates the skewness taken on derivative blower pressure, with epoch mean.

In some implementations, the internal microphone of the respiratory therapy system can detect variability in noise levels and/or acoustic characteristics associated with mask leak. For example, leak detection can be performed based on (i) sound level features, and/or (ii) spectral features (e.g., ratio of energy content in various frequency bands).

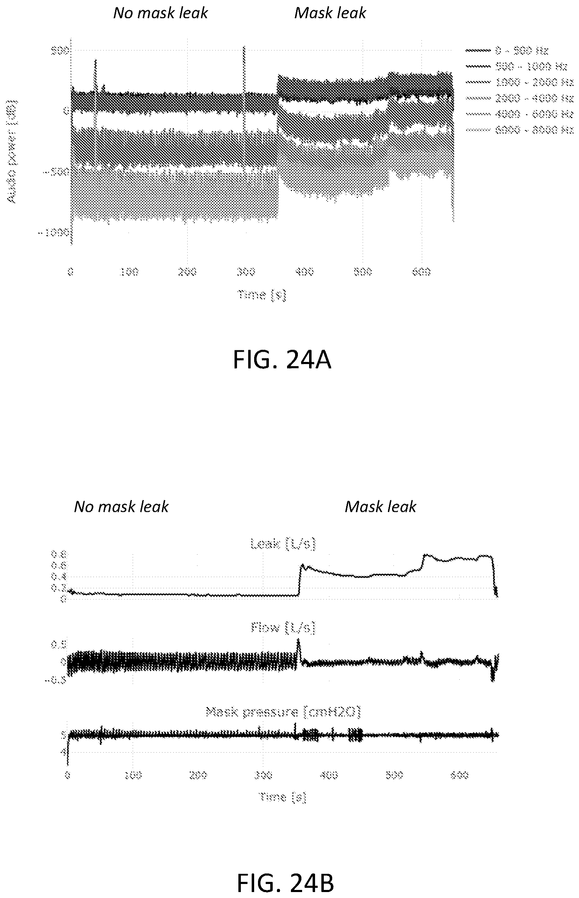

FIG. 24A acoustic power levels over a time period of no mask leak and a time period of mask leak. The acoustic data generated by the microphone 140 (FIG. 1) detects variability in noise levels and acoustic characteristics or patterns associated with the acoustic signatures corresponding to the five-minute time period of no mask leak and the five-minute time period of mask leak within the respiratory therapy system 120. As shown in FIG. 24A, a leak in the user interface 124 (mask leak) can be detected from the plotted acoustic data over the time periods, based on sound level features and/or spectral features such as the acoustic energy ratio in the different frequency bands (between about 0 and about 8KHz in the plot of FIG. 24A).

FIG. 24B a comparative graphical representation of leak rate, flow rate, and mask pressure, over the time period of no mask leak and the time period of mask leak of FIG. 24A. As indicated by FIG. 24B, the detection of mask leak in the user interface 124 from the acoustic data of FIG. 24A correlates with an indication of mask leak in the user interface 124 from the data on pressure, flow rate, and leak rate in the user interface 124 over the same five-minute time period of no mask leak and the same five-minute time period of mask leak in the user interface 124.

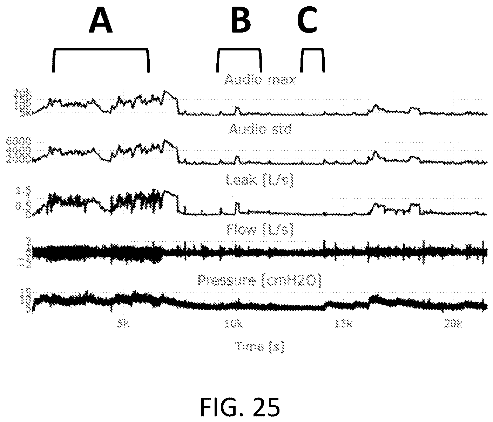

FIG. 25 illustrates a comparative graphical representation of maximum value of acoustic intensity, standard deviation of acoustic intensity, leak rate (measured in liters per second), flow rate (measured in liters per second), and mask pressure (measured in cm $H_2O$) over a time period of more than 20,000 seconds, during which leaks occur in the respiratory therapy system. Acoustic intensity is one of the parameters determined from the acoustic data in FIG. 25 generated by the microphone positioned with the respiratory therapy device.

Statistical data associated with the parameter such as, but not limited to, standard deviation of acoustic intensity, maximum value of acoustic intensity, and percentiles of acoustic intensity are extracted from short windows (e.g., 0.1 second) of acoustic data sampled over predetermined time intervals (e.g., 1 second) throughout overlapping or non-overlapping windows of time within the time period. The statistical data collected over the time period is then low-pass filtered (for example, by a rolling average or applying a digital filter such as a finite impulse response (FIR), or an infinite impulse response (IIR)). Occurrence of a leak is determined based on whether the parameter satisfies a condition (for example, being above a predetermined threshold) as described herein.

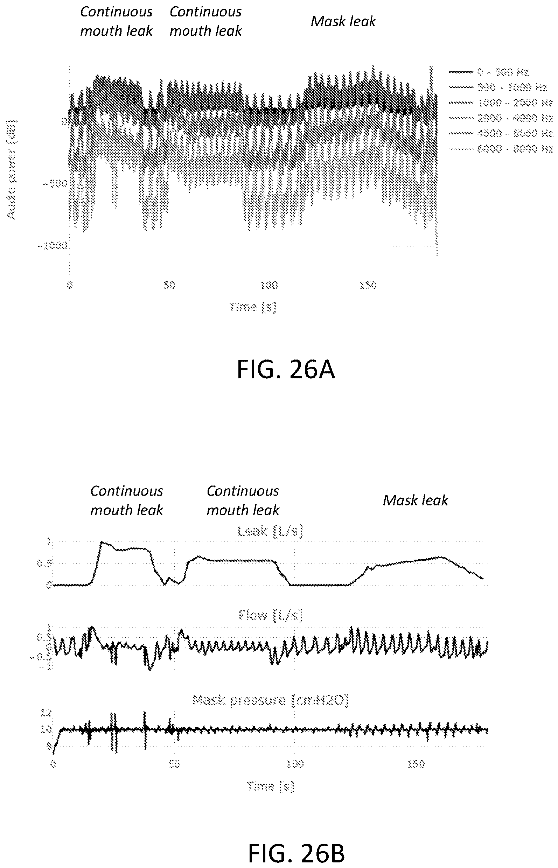

As shown in FIG. 25, the statistical data is plotted with the mask pressure, flow rate, and leak rate over the time period. The comparative graphical representation in FIG. 25 shows a correlation among the statistical data for acoustic intensity, flow rate, mask pressure, and the leak rate to indicate no leak (inset C), as well as high levels of leak (inset A); and relatively low levels of leak (inset B) commensurate with typical errors associated with inaccurate estimation of impedance of airflow within the respiratory therapy system. In some implementations, another parameter such as acoustic energy ratios in different frequency bands, may be used to extract statistical data from acoustic data generated by the microphone, as described with respect to FIGS. 24A-24B and FIGS. 26A-26B.

FIG. 26A acoustic power levels over a time period during which different types of leak occur, where the leaks can be distinguished based on location of the leak within the respiratory therapy system. The acoustic data generated by the microphone may have acoustic features having different acoustic characteristics depending on the type of leak. Different conditions may have to be satisfied (for example, different thresholds may be applied to the parameters in the acoustic data) depending on the type of leak.

As shown in FIG. 26A, a mask leak is indicated by a distinct acoustic signature than a mouth leak (CML or VML), based on sound level features and spectral features such as the acoustic energy ratio in the different frequency bands (between about 0 and about 8KHz in the plot of FIG. 26A). The distribution of acoustic energy across the different frequency bands in FIG. 26A illustrates a clear difference between the two types of leaks as indicated by a higher acoustic energy content in the lower frequency bands for mask leak and in the higher frequency bands for mouth leak.

FIG. 26B comparative graphical representation of leak rate, flow rate, and mask pressure, over the time period of FIG. 26A. As indicated by FIG. 26B, the detection of mask leak and mouth leak (CML or VML) from the acoustic data of FIG. 26A clearly correlates with corresponding indications of mask leak and mouth leak, from the data on mask pressure, flow rate, and leak rate over the same time period of FIG. 26A.

**Exemplary Embodiments**

1. A method for determining a mouth leak status associated with a user of a respiratory device, comprising:

receiving airflow data associated with the user of the respiratory device, the respiratory device being configured to supply pressurized air to an airway of the user during a therapy session, the airflow data including pressure data and/or flow rate data;
analyzing the airflow data associated with the user; and
based at least in part on the analysis, determining the mouth leak status associated with the user, the mouth leak status being indicative of whether or not air is leaking from a mouth of the user.

2. The method of embodiment 1, wherein the analyzing the airflow data associated with the user includes processing the airflow data to identify one or more features that distinguish mouth leak from (i) normal respiration during therapy and/or (ii) other types of unintentional leak.

3. The method of embodiment 2, wherein the one or more features includes a covariance between leak and ventilation, a time the covariance holds above a threshold, a normalized ventilation, an unintentional leak variability, a normalized respiration rate, a respiration rate variability, or any combination thereof.

4. The method of embodiment 2 or embodiment 3, wherein the one or more features are computed on a user flow rate signal, a mask pressure signal, a blower flow rate signal, a blower pressure signal, or any combination thereof; wherein the one or more features include, for each signal, (i) a frame area, (ii) a breath area, (iii) a complement to the breath area, (iv) a ratio of the breath area over the frame area, (v) a ratio of the breath area over the complement to the breath area, (vi) a skewness of the signal, (vii) a kurtosis of the signal, (viii) a first derivative of the skewness, (ix) a first derivative of the kurtosis, (x) a second derivative of the skewness, (xi) a second derivative of the kurtosis, or (xii) any combination thereof.

5. The method of any one of embodiments 2 to 4, wherein the one or more features includes a minimum pressure, a maximum pressure, a pressure skewness, a pressure kurtosis, a pressure power spectral density, a flow rate range, a minimum flow rate, a maximum flow rate, a flow skewness, a flow kurtosis, a flow sub-area ratio, or any combination thereof.

6. The method of embodiment 5, wherein the minimum pressure is associated with an end of the inhalation portion, a beginning of the exhalation portion, or both.

7. The method of any one of embodiments 2 to 6, wherein the one or more features are calculated over 1, 2, 3, 4, 5, 6, 7, or 8 adjacent breaths.

8. The method of any one of embodiments 2 to 7, wherein the one or more features are calculated over about 30 seconds.

9. The method of any one of embodiments 5 to 8, wherein the flow sub-area ratio is calculated by dividing a first sub-area from a second sub-area, the first sub-area being a portion of a flow expiratory area, the second sub-area being the flow expiratory area, wherein the flow expiratory area is delimited by a flow expiratory curve and zero flow rate, wherein the portion of the flow expiratory area is delimited by the flow expiratory curve and a flow threshold level.

10. The method of embodiment 9, wherein the flow threshold level is calculated by adding a predetermined percentage of the flow rate range to the minimum flow rate.

11. The method of embodiment 10, wherein the predetermined percentage is 25%.

12. The method of any one of embodiments 9 to 11, wherein the mouth leak status is determined based, at least in part, on the pressure range, a detrended minimum pressure, and the flow sub-area ratio.

13. The method of any one of embodiments 9 to 12, wherein the mouth leak status is determined based, at least in part, on an output from a logistic regression model, and wherein the logistic regression model can be calculated by:

$$p = \frac{1}{1 + e^{-(b + \alpha_1 x_1 + \alpha_2 x_2 + \alpha_3 x_3)}}$$

14. The method of embodiment 13, wherein the output from the logistic regression model greater than or equal to a threshold is indicative of the mouth leak status being valve-like mouth leak or continuous mouth leak.

15. The method of embodiment 14, wherein the threshold is 0.6.

16. The method of any one of embodiments 2 to 15, further comprising determining an operational mode of the respiratory device, wherein the one or more features are determined based at least in part on the determined operational mode.

17. The method of embodiment 16, wherein the operational mode is CPAP, APAP, or BiPAP.

18. The method of embodiment 16 or embodiment 17, wherein the one or more features are determined based at least in part on removing an Expiratory Pressure Relief (EPR) component in the pressure data.

19. The method of any one of embodiments 2 to 18, wherein the one or more features are associated with a first breath.

20. The method of embodiment 19, further comprising identifying, within the received airflow data, the first breath of the user, the first breath having an inhalation portion and an exhalation portion.

21. The method of embodiment 20, wherein the identifying the first breath includes identifying a beginning of the first breath, an end of the first breath, or both.

22. The method of any one of embodiments 1 to 21, wherein the mouth leak status is (i) no mouth leak, (ii) valve-like mouth leak, or (iii) continuous mouth leak.

23. The method of any one of embodiments 1 to 22, wherein the pressurized air supplied to the airway of the user during the therapy session is between 4 cmH$_2$O to 20 cmH$_2$O.

24. The method of any one of embodiments 1 to 23, further comprising calculating a therapy score or AHI score based at least in part on the determined mouth leak status.

25. The method of embodiment 24, further comprising:

receiving, from a sensor coupled to the respiratory device, sensor data associated with the user during the therapy session, the sensor data being indicative of a number of sleep-disordered breathing events during the therapy session;
correlating the mouth leak status with the sensor data to output one or more false positive sleep-disordered breathing events;
subtracting the one or more false positive sleep-disordered breathing events from the number of sleep-disordered breathing events to output a modified number of sleep-disordered breathing events; and
calculating the therapy score based, at least in part, on the modified number of sleep-disordered breathing events.

26. The method of any one of embodiments 1 to 25, wherein the mouth leak status includes a duration of mouth leak, a severity of mouth leak, or both; and wherein the method further comprises decreasing a sleep score or therapy score based, at least in part, on the duration of mouth leak, the severity of mouth leak, or both.

27. The method of any one of embodiments 1 to 26, further comprising:

providing control signals to the respiratory device; and
responsive to the mouth leak status, adjusting pressure settings of the respiratory device, the pressure settings being associated with the pressurized air supplied to the airway of the user.

28. The method of embodiment 27, further comprising:

analyzing the airflow data associated with the user to determine that the user is exhaling; and

responsive to the determination that the user is exhaling, reducing a pressure of the pressurized air to the airway of the user during the exhaling of the user.

29. The method of embodiment 28, wherein the reducing the pressure of the pressurized air includes increasing an Expiratory Pressure Relief (EPR) level associated with the respiratory device.

30. The method of any one of embodiments 1 to 29, further comprising:

providing control signals to a humidifier coupled to the respiratory device, the humidifier being configured to introduce moisture to the pressurized air supplied to the airway of the user; and
responsive to the mouth leak status, adjusting humidification settings associated with the humidifier such that more moisture is introduced into the pressurized air supplied to the airway of the user.

31. The method of embodiment 30, further comprising releasing a portion of a decongestant into the moisture to be introduced into the pressurized air for the adjusting the humidification settings.

32. The method of any one of embodiments 1 to 31, further comprising:

providing control signals to a smart pillow; and
responsive to the mouth leak status, adjusting the smart pillow such that the smart pillow urges the user to change position of the user's head.

33. The method of any one of embodiments 1 to 32, further comprising:

providing control signals to a smart bed or a smart mattress; and
responsive to the mouth leak status, adjusting the smart bed or the smart mattress such that the smart bed or the smart mattress urges the user to change position of the user's body.

34. The method of any one of embodiments 1 to 33, further comprising:

providing control signals to a wearable sensor, the wearable sensor being couplable to a body part of the user; and
responsive to the mouth leak status, adjusting the wearable sensor such that the wearable sensor stimulates a neck or a jaw of the user to close the user's mouth.

35. The method of any one of embodiments 1 to 34, further comprising responsive to the mouth leak status, causing a notification to be provided to the user via an electronic device, such that the user is alerted of the mouth leak status.

36. The method of embodiment 35, wherein the electronic device is an electronic display device and the providing the notification includes displaying, on the electronic display device, a message.

37. The method of embodiment 35 to embodiment 36, wherein the notification includes a reminder for the user to (i) close his/her mouth during the therapy session, (ii) moisturize lips before a next therapy session, or (iii) both (i) and (ii).

38. The method of any one of embodiments 35 to 37, wherein the notification includes an instruction and/or recommendation to the user (i) to use a different mask, (ii) to wake up, (iii) that the user is having a mouth leak, or a combination thereof.

39. The method of any one of embodiments 35 to 38, wherein the electronic device includes a speaker and the providing the notification includes playing, via the speaker, sound.

40. The method of embodiment 39, wherein the sound is loud enough to wake up the user.

41. The method of any one of embodiments 1 to 40, wherein the mouth leak status includes a mouth leak score for the therapy session.

42. The method of embodiment 41, wherein the mouth leak score is determined based, at least in part, on a percentage of mouth leak during the therapy session, a mouth leak peak volume, a mouth leak total volume, or a combination thereof.

43. The method of embodiment 41 or embodiment 42, further comprising:

receiving, from a user device, user input data indicative of subjective feedback associated with the user; and determining the mouth leak score based, at least in part, on the user input data.

44. The method of any one of embodiments 1 to 43, further comprising:

receiving sleep stage data associated with the user during the therapy session;
determining a sleep stage based at least in part on the sleep stage data; and
associate the mouth leak status with the sleep stage.

45. The method of embodiment 44, wherein the sleep stage includes wake, drowsy, sleep, light sleep, deep sleep, N1 sleep, N2 sleep, N3 sleep, REM sleep, sleep stage fragmentation, or a combination thereof.

46. The method of embodiment 44 or embodiment 45, further comprising:
causing an indication to be displayed on a display device, the indication including a separate mouth leak status per sleep stage.

47. A system comprising:

a control system including one or more processors; and
a memory having stored thereon machine readable instructions;
wherein the control system is coupled to the memory, and the method of any one of embodiments 1 to 46 is implemented when the machine executable instructions in the memory are executed by at least one of the one or more processors of the control system.

48. A system for determining a mouth leak status associated with a user of a respiratory device, the system including a control system configured to implement the method of any one of embodiments 1 to 46.

49. A computer program product comprising instructions which, when executed by a computer, cause the computer to carry out the method of any one of embodiments 1 to 46.

50. The computer program product of embodiment 49, wherein the computer program product is a non-transitory computer readable medium.

51. A method for determining a mouth leak status, comprising:

receiving, from a microphone, first acoustic data associated with a user of a respiratory device, the respiratory device being configured to supply pressurized air to an airway of the user during a sleep session;
analyzing the first acoustic data associated with the user; and
determining the mouth leak status based, at least in part, on the analysis of the first acoustic data, the mouth leak status being indicative of air leaking from a mouth of the user.

52. The method of embodiment 51, wherein the determining the mouth leak status includes distinguishing mouth leak from user interface leak.

53. The method of embodiment 51 or embodiment 52, further comprising comparing the first acoustic data with predetermined data indicative of a negative mouth leak status for the analyzing the first acoustic data.

54. The method of embodiment 53, wherein the predetermined data includes simulated data, historical data, or both.

55. The method of any one of embodiments 51 to 54, wherein the analyzing the first acoustic data is based, at least in part, on a Cepstrum analysis, an autocepstrum analysis, an auto-correlation analysis, a spectral analysis, or any combination thereof.

56. The method of embodiment 55, wherein the spectral analysis includes a fast Fourier transform (FFT) with a sliding window, a spectrogram, a neutral network, a short time Fourier transform (STFT), a wavelet-based analysis, or any combination thereof.

57. The method of any one of embodiments 51 to 56, further comprising processing the first acoustic data to identify a plurality of features for the analyzing the first acoustic data.

58. The method of embodiment 57, wherein the plurality of features includes (i) a change in spectral signature, (ii) a change in frequency, (iii) a change in amplitude, (iv) a change in energy distribution across frequency bands, or (v) any combination thereof.

59. The method of any one of embodiments 51 to 58, wherein the microphone is an integrated microphone coupled to (i) a conduit of the respiratory device, (ii) a circuit board of the respiratory device, (iii) a connector of a respiratory system having the respiratory device, (iv) a user interface of the respiratory system, or (v) any other component of the respiratory system.

60. The method of embodiment 59, wherein the microphone is in fluid communication and/or acoustic communication with an air pathway fluidly coupled with an airway of the user.

61. The method of any one of embodiments 51 to 60, further comprising:

receiving, from an external microphone, second acoustic data associated the user of the respiratory device during the sleep session;
analyzing the second acoustic data associated with the user; and
determining the mouth leak status based, at least in part, on both the analysis of the first acoustic data and the analysis of the second acoustic data.

62. The method of any one of embodiments 51 to 61, further comprising:

receiving, from a physiological sensor, physiological data associated with the user during the sleep session;
analyzing the physiological data to determine cardiogenic oscillations of the user; and
determining the mouth leak status based, at least in part, on both the analysis of the first acoustic data and the cardiogenic oscillations of the user.

63. A method for determining an optimal inhalation pressure and an optimal exhalation pressure for a user of a respiratory device, comprising:

receiving, from a microphone, acoustic data during a plurality of sleep sessions, the microphone being associated with the user of the respiratory device, the respiratory device being configured to supply pressurized air to an airway of the user, the acoustic data including inhalation acoustic data and exhalation acoustic data;
receiving pressure data associated with the pressurized air supplied to the airway of the user during the plurality of sleep sessions, the pressure data including inhalation pressure data and exhalation pressure data;
analyzing the acoustic data to determine a mouth leak status of the user for each sleep session of the plurality of sleep sessions, the mouth leak status being indicative of air leaking from a mouth of the user; and
determining, based at least in part on (i) the mouth leak status of the user for each sleep session of the plurality of sleep sessions and (ii) the pressure data, the optimal inhalation pressure and the optimal exhalation pressure for the user.

64. The method of embodiment 63, further comprising adjusting pressure settings of the respiratory device based at least in part on the optimal inhalation pressure and the optimal exhalation pressure for the user.

65. The method of embodiment 63 or embodiment 64, further comprising:

receiving, from the microphone, subsequent acoustic data during a subsequent sleep session;

receiving the optimal inhalation pressure and the optimal exhalation pressure as subsequent pressure data for the subsequent sleep session; and

repeating the analyzing and the determining to update the optimal inhalation pressure and the optimal exhalation pressure for the user.

## Alternative Implementations

Alternative Implementation 1. A method for determining a mouth leak status, comprising: receiving, from a microphone, first acoustic data associated with a user of a respiratory device, the respiratory device being configured to supply pressurized air to an airway of the user during a sleep session; analyzing the first acoustic data associated with the user; and determining the mouth leak status based, at least in part, on the analysis of the first acoustic data, the mouth leak status being indicative of air leaking from a mouth of the user.

Alternative Implementation 2. The method of Alternative Implementation 1, further comprising comparing the first acoustic data with predetermined data indicative of a negative mouth leak status for the analyzing the first acoustic data.

Alternative Implementation 3. The method of Alternative Implementation 2, wherein the predetermined data includes simulated data, historical data, or both.

Alternative Implementation 4. The method of any one of Alternative Implementations 1 to 3, wherein the analyzing the first acoustic data is based, at least in part, on a Cepstrum analysis, an autocepstrum analysis, an auto-correlation analysis, a spectral analysis, or any combination thereof.

Alternative Implementation 5. The method of Alternative Implementation 4, wherein the spectral analysis includes a fast Fourier transform (FFT) with a sliding window, a spectrogram, a neutral network, a short time Fourier transform (STFT), a wavelet-based analysis, or any combination thereof.

Alternative Implementation 6. The method of any one of Alternative Implementations 1 to 5, further comprising processing the first acoustic data to identify a plurality of features for the analyzing the first acoustic data.

Alternative Implementation 7. The method of Alternative Implementation 6, wherein the plurality of features includes (i) a change in spectral signature, (ii) a change in frequency, (iii) a change in amplitude, or (iv) any combination thereof.

Alternative Implementation 8. The method of any one of Alternative Implementations 1 to 7, wherein the microphone is an integrated microphone coupled to (i) a conduit of the respiratory device, (ii) a circuit board of the respiratory device, (iii) a connector of a respiratory system having the respiratory device, (iv) a user interface of the respiratory system, or (v) any other component of the respiratory system.

Alternative Implementation 9. The method of any one of Alternative Implementations 1 to 8, further comprising: receiving, from an external microphone, second acoustic data associated the user of the respiratory device during the sleep session; analyzing the second acoustic data associated with the user; and determining the mouth leak status based, at least in part, on both the analysis of the first acoustic data and the analysis of the second acoustic data.

Alternative Implementation 10. The method of any one of Alternative Implementations 1 to 9, further comprising: receiving, from a flow sensor, airflow data associated with the user of the respiratory device during the sleep session; analyzing the airflow data associated with the user; and determining the mouth leak status based, at least in part, on both the analysis of the first acoustic data and the analysis of the airflow data of the user.

Alternative Implementation 11. The method of any one of Alternative Implementations 1 to 10, further comprising: receiving, from a physiological sensor, physiological data associated with the user during the sleep session; analyzing the physiological data to determine cardiogenic oscillations of the user; and determining the mouth leak status based, at least in part, on both the analysis of the first acoustic data and the cardiogenic oscillations of the user.

Alternative Implementation 12. The method of any one of Alternative Implementations 1 to 11, further comprising: receiving, from a camera, image data associated with the user during the sleep session; analyzing the image data to determine sleep-related parameters associated with the user; and determining the mouth leak status based, at least in part, on both the analysis of the first acoustic data and the sleep-related parameters associated with the user.

Alternative Implementation 13. The method of any one of Alternative Implementations 1 to 12, further comprising: calculating an Apnea-Hypopnea Index (AHI) score based, at least in part, on the mouth leak status.

Alternative Implementation 14. The method of Alternative Implementation 13, wherein, for the calculating the AHI score, the control system is configured to execute the machine-readable instructions to: receiving, from a sensor coupled to the respiratory device, sensor data associated with the user during the sleep session, the sensor data being indicative of a number of sleep-disordered breathing events during the sleep session; correlating the mouth leak status with the sensor data to output one or more false positive sleep-disordered breathing events; subtracting the one or more false positive sleep-disordered breathing events from the number of sleep-disordered breathing events to output a modified number of sleep-disordered breathing events; and calculating the AHI score based, at least in part, on the modified number of sleep-disordered breathing events.

Alternative Implementation 15. The method of any one of Alternative Implementations 1 to 14, wherein the mouth leak status includes a duration of mouth leak, a severity of mouth leak, or both; and wherein the method further comprises decreasing a sleep score or therapy score based, at least in part, on the duration of mouth leak, the severity of mouth leak, or both.

Alternative Implementation 16. The method of any one of Alternative Implementations 1 to 15, further comprising: providing control signals to the respiratory device; and responsive to the mouth leak status, adjusting pressure settings of the respiratory device, the pressure settings being associated with the pressurized air supplied to the airway of the user.

Alternative Implementation 17. The method of Alternative Implementation 16, further comprising: analyzing the first acoustic data associated with the user to determine that the user is exhaling; and responsive to the determination that the user is exhaling, reducing a pressure of the pressurized air to the airway of the user during the exhaling of the user.

Alternative Implementation 18. The method of Alternative Implementation 17, wherein the reducing the pressure of the pressurized air includes increasing an Expiratory Pressure Relief (EPR) level associated with the respiratory device.

Alternative Implementation 19. The method of any one of Alternative Implementations 1 to 18, further comprising: providing control signals to a humidifier coupled to the respiratory device, the humidifier being configured to introduce moisture to the pressurized air supplied to the airway of the user; and responsive to the mouth leak status, adjusting humidification settings associated with the humidifier such that more moisture is introduced into the pressurized air supplied to the airway of the user.

Alternative Implementation 20. The method of Alternative Implementation 19, further comprising releasing a portion of a decongestant into the moisture to be introduced into the pressurized air for the adjusting the humidification settings.

Alternative Implementation 21. The method of any one of Alternative Implementations 1 to 20, further comprising: providing control signals to a smart pillow; and responsive to the mouth leak status, adjusting the smart pillow such that the smart pillow urges the user to change position of the user's head.

Alternative Implementation 22. The method of any one of Alternative Implementations 1 to 21, further comprising: providing control signals to a smart bed or a smart mattress; and responsive to the mouth leak status, adjusting the smart bed or the smart mattress such that the smart bed or the smart mattress urges the user to change position of the user's body.

Alternative Implementation 23. The method of any one of Alternative Implementations 1 to 22, further comprising: providing control signals to a wearable sensor, the wearable sensor being couplable to a body part of the user; and responsive to the mouth leak status, adjusting the wearable sensor such that the wearable sensor stimulates a neck or a jaw of the user to close the user's mouth.

Alternative Implementation 24. The method of any one of Alternative Implementations 1 to 23, further comprising: responsive to the mouth leak status, causing a notification to be provided to the user via an electronic device, such that the user is alerted of the mouth leak status.

Alternative Implementation 25. The method of Alternative Implementation 24, wherein the electronic device is an electronic display device and the providing the notification includes displaying, on the electronic display device, a message.

Alternative Implementation 26. The method of Alternative Implementation 25, wherein the electronic display device is a mobile phone.

Alternative Implementation 27. The method of any one of Alternative Implementations 24 to 26, wherein the notification includes a reminder for the user to (i) close his/her mouth during the sleep session, (ii) moisturize lips before a next sleep session, or (iii) both (i) and (ii).

Alternative Implementation 28. The method of any one of Alternative Implementations 24 to 27, wherein the notification includes an instruction and/or recommendation to the user (i) to use a different mask, (ii) to wake up, (iii) that the user is having a mouth leak, or any combination thereof.

Alternative Implementation 29. The method of any one of Alternative Implementations 24 to 28, wherein the electronic device includes a speaker and the providing the notification includes playing, via the speaker, sound.

Alternative Implementation 30. The method of Alternative Implementation 29, wherein the sound is loud enough to wake up the user.

Alternative Implementation 31. The method of any one of Alternative Implementations 1 to 30, wherein the mouth leak status includes a mouth leak score for the sleep session.

Alternative Implementation 32. The method of Alternative Implementation 31, wherein the mouth leak score is determined based, at least in part, on a percentage of mouth leak during the sleep session, a mouth leak peak volume, a mouth leak total volume, or any combination thereof.

Alternative Implementation 33. The method of Alternative Implementation 31 or Alternative Implementation 32, further comprising: receiving, from a user device, user input data indicative of subjective feedback associated with the user; and determining the mouth leak score based, at least in part, on the user input data.

Alternative Implementation 34. The method of any one of Alternative Implementations 1 to 33, further comprising:

receiving sleep stage data associated with the user during the sleep session; determining a sleep stage based at least in part on the sleep stage data; and associate the mouth leak status with the sleep stage.

Alternative Implementation 35. The method of Alternative Implementation 34, wherein the sleep stage includes wake, drowsy, sleep, light sleep, deep sleep, N1 sleep, N2 sleep, N3 sleep, REM sleep, sleep stage fragmentation, or any combination thereof.

Alternative Implementation 36. The method of Alternative Implementation 34 or Alternative Implementation 35, further comprising: causing an indication to be displayed on a display device, the indication including a separate mouth leak status per sleep stage.

Alternative Implementation 37. A method for outputting a mouth leak status for a user of a respiratory device, comprising: receiving, from a microphone, acoustic data associated with the user of the respiratory device, the respiratory device being configured to supply pressurized air to an airway of the user during a sleep session; and processing, using a machine learning algorithm, the acoustic data to output the mouth leak status for the user, the mouth leak status being indicative of air leaking from a mouth of the user.

Alternative Implementation 38. A method for determining an optimal inhalation pressure and an optimal exhalation pressure for a user of a respiratory device, comprising: receiving, from a microphone, acoustic data during a plurality of sleep sessions, the microphone being associated with the user of the respiratory device, the respiratory device being configured to supply pressurized air to an airway of the user, the acoustic data including inhalation acoustic data and exhalation acoustic data; receiving pressure data associated with the pressurized air supplied to the airway of the user during the plurality of sleep sessions, the pressure data including inhalation pressure data and exhalation pressure data; analyzing the acoustic data to determine a mouth leak status of the user for each sleep session of the plurality of sleep sessions, the mouth leak status being indicative of air leaking from a mouth of the user; and determining, based at least in part on (i) the mouth leak status of the user for each sleep session of the plurality of sleep sessions and (ii) the pressure data, the optimal inhalation pressure and the optimal exhalation pressure for the user.

Alternative Implementation 39. The method of Alternative Implementation 12 or Alternative Implementation 38, wherein the pressure data is received from a pressure sensor coupled to the respiratory device.

Alternative Implementation 40. The method of Alternative Implementation 12, 38, or 39, wherein the pressure data is received from a pressure sensor external to the respiratory device.

Alternative Implementation 41. The method of Alternative Implementation 12, 38, 39, or 40, wherein the pressure data is received from the respiratory device.

Alternative Implementation 42. The method of any one of Alternative Implementations 38 to 41, further comprising: adjusting pressure settings of the respiratory device based at least in part on the optimal inhalation pressure and the optimal exhalation pressure for the user.

Alternative Implementation 43. The method of any one of Alternative Implementations 38 to 42, further comprising: receiving, from the microphone, subsequent acoustic data during a subsequent sleep session; receiving the optimal inhalation pressure and the optimal exhalation pressure as subsequent pressure data for the subsequent sleep session; and repeating the analyzing and the determining to update the optimal inhalation pressure and the optimal exhalation pressure for the user.

Alternative Implementation 44. A method for determining an estimated mouth leak status, comprising: receiving, from a microphone, acoustic data during a plurality of sleep sessions, the microphone being associated with a user of a respiratory device, the respiratory device being configured to supply pressurized air to an airway of the user; receiving, from a sensor, physiological data associated with the user for each sleep session of the plurality of sleep sessions; analyzing the acoustic data to determine a mouth leak status of the user for each sleep session of the plurality of sleep sessions, the mouth leak status being indicative of air leaking from a mouth of the user; and training a machine learning algorithm with (i) the mouth leak status of the user for each sleep session of the plurality of sleep sessions and (ii) the physiological data, such that the machine learning algorithm is configured to: receive as an input current physiological data associated with a current sleep session; and determine as an output the estimated mouth leak status for the current sleep session.

Alternative Implementation 45. The method of Alternative Implementation 44, wherein the microphone and the sensor are the same.

Alternative Implementation 46. The method of Alternative Implementation 11 or Alternative Implementation 44, wherein the physiological data generated by the sensor includes breath alcohol data, blood alcohol data, blood pressure data, blood glucose data, congestion data, occlusion data, body temperature data, heart rate data, movement data, respiration data, sleep stage data, mask data, $CO_2$ level data, or any combination thereof.

Alternative Implementation 47. The method of Alternative Implementation 46, wherein the respiration data includes a respiration rate, a respiration shape, or both.

Alternative Implementation 48. The method of any one of Alternative Implementations 38 to 47, further comprising: receiving, as the input to the machine learning algorithm, the current physiological data during the current sleep session; generating, as the output of the machine learning algorithm, the estimated mouth leak status for the current sleep session; and adjusting, based at least in part on the estimated mouth leak status, pressure settings of the respiratory device.

Alternative Implementation 49. The method of any one of Alternative Implementations 38 to 48, further comprising: receiving, as the input to the machine learning algorithm, the current physiological data prior to the next sleep session; generating, as the output of the machine learning algorithm, the estimated mouth leak status for the next sleep session; determining, based at least in part on the estimated mouth leak status, a recommended adjustment for displaying on a user device.

Alternative Implementation 50. The method of Alternative Implementation 49, wherein the recommended adjustment includes (i) adjusting pressure settings of the respiratory device, the pressure settings being associated with the pressurized air supplied to the airway of the user; (ii) adjusting humidification settings of a humidifier coupled to the respiratory device, the humidifier being configured to introduce moisture to the pressurized air supplied to the airway of the user; (iii) recommending a mask type for the respiratory device, (iv) recommending a sleep position for the user, (v) recommending a chin strap for the user; (vi) recommending a nasal cradle cushion for the user; (vii) any combination thereof.

Alternative Implementation 51. A system comprising: a control system including one or more processors; and a memory having stored thereon machine readable instructions; wherein the control system is coupled to the memory, and the method of any one of Alternative Implementations 1 to 50 is implemented when the machine executable instructions in the memory are executed by at least one of the one or more processors of the control system.

Alternative Implementation 52. A system comprising a control system configured to implement the method of any one of Alternative Implementations 1 to 50.

Alternative Implementation 53. A computer program product comprising instructions which, when executed by a computer, cause the computer to carry out the method of any one of Alternative Implementations 1 to 50.

Alternative Implementation 54. The computer program product of Alternative Implementation 53, wherein the computer program product is a non-transitory computer readable medium.

Alternative Implementation 55. A method for determining a mouth leak status associated with a user of a respiratory device, comprising: receiving airflow data associated with the user of the respiratory device, the respiratory device being configured to supply pressurized air to an airway of the user during a therapy session, the airflow data including pressure data; analyzing the airflow data associated with the user; and based at least in part on the analysis, determining the mouth leak status associated with the user, the mouth leak status being indicative of whether or not air is leaking from a mouth of the user.

Alternative Implementation 56. The method of Alternative Implementation 55, wherein the airflow data further includes flow rate data.

Alternative Implementation 57. The method of Alternative Implementation 56, wherein the flow rate data is received from a flow rate sensor associated with the respiratory device.

Alternative Implementation 58. The method of Alternative Implementation 57, wherein the flow rate sensor is integrated in the respiratory device, coupled to the respiratory device, or both.

Alternative Implementation 59. The method of any one of Alternative Implementations 55 to 58, wherein the pressure data is received from a pressure sensor associated with the respiratory device.

Alternative Implementation 60. The method of Alternative Implementation 59, wherein the pressure sensor is integrated in the respiratory device, coupled to the respiratory device, or both.

Alternative Implementation 61. The method of any one of Alternative Implementations 55 to 60, further comprising: identifying, within the received airflow data, a first breath cycle of the user, the first breath cycle having an inhalation portion and an exhalation portion.

Alternative Implementation 62. The method of Alternative Implementation 61, wherein a length of the first breath cycle of the user is about five seconds.

Alternative Implementation 63. The method of Alternative Implementation 55, wherein the identifying the first breath cycle includes identifying a beginning of the first breath, an end of the first breath, or both.

Alternative Implementation 64. The method of any one of Alternative Implementations 61 to 63, wherein the analyzing the airflow data associated with the user includes processing the airflow data to identify one or more features associated with the first breath cycle.

Alternative Implementation 65. The method of Alternative Implementation 64, wherein the one or more features includes a minimum pressure, a maximum pressure, a pressure skewness, a pressure kurtosis, a pressure power spectral density, a flow rate range, a minimum flow rate, a maximum flow rate, a flow skewness, a flow kurtosis, a flow sub-area ratio, or any combination thereof.

Alternative Implementation 66. The method of Alternative Implementation 65, wherein boundaries of the pressure range are defined by the minimum pressure and the maximum pressure.

Alternative Implementation 67. The method of Alternative Implementation 65 or Alternative Implementation 66, wherein the minimum pressure is associated with an end of the inhalation portion, a beginning of the exhalation portion, or both.

Alternative Implementation 68. The method of any one of Alternative Implementations 65 to 67, wherein the one or more features associated with the first breath cycle are calculated over 1, 2, 3, 4, 5, 6, 7, or 8 adjacent breath cycles.

Alternative Implementation 69. The method of Alternative Implementation 68, wherein the one or more features associated with the first breath cycle are calculated over about 30 seconds.

Alternative Implementation 70. The method of any one of Alternative Implementations 65 to 69, wherein the flow sub-area ratio is calculated by dividing a first sub-area from a second sub-area, the first sub-area being a portion of a flow expiratory area, the second sub-area being the flow expiratory area, wherein the flow expiratory area is delimited by a flow expiratory curve and zero flow rate, wherein the portion of the flow expiratory area is delimited by the flow expiratory curve and a flow threshold level.

Alternative Implementation 71. The method of Alternative Implementation 70, wherein the flow threshold level is calculated by adding a predetermined percentage of the flow rate range to the minimum flow rate.

Alternative Implementation 72. The method of Alternative Implementation 71, wherein the predetermined percentage is 25%.

Alternative Implementation 73. The method of any one of Alternative Implementations 70 to 72, wherein the flow threshold level is adjusted based at least in part on further analyzing the airflow data associated with the user.

Alternative Implementation 74. The method of any one of Alternative Implementations 70 to 73, wherein the mouth leak status is determined based, at least in part, on the pressure range, a detrended minimum pressure, and the flow sub-area ratio.

Alternative Implementation 75. The method of any one of Alternative Implementations 70 to 74, wherein the mouth leak status is determined based, at least in part, on an output from a logistic regression model, and wherein the logistic regression model can be calculated by:

$$p = \frac{1}{1 + e^{-(b + \alpha_1 x_1 + \alpha_2 x_2 + \alpha_3 x_3)}}$$

Alternative Implementation 76. The method of Alternative Implementation 75, wherein the output from the logistic regression model greater than or equal to a threshold is indicative of the mouth leak status being valve-like mouth leak or continuous mouth leak.

Alternative Implementation 77. The method of Alternative Implementation 76, wherein the threshold is 0.6.

Alternative Implementation 78. The method of any one of Alternative Implementations 65 to 77, further comprising: determining an operational mode of the respiratory device.

Alternative Implementation 79. The method of Alternative Implementation 78, wherein the operational mode is CPAP, APAP, or BiPAP.

Alternative Implementation 80. The method of any one of Alternative Implementations 78 to 79, wherein the one or more features are determined based at least in part on the determined operational mode.

Alternative Implementation 81. The method of any one of Alternative Implementations 78 to 80, wherein the one or more features are determined based at least in part on removing an Expiratory Pressure Relief (EPR) component in the pressure data.

Alternative Implementation 82. The method of any one of Alternative Implementations 55 to 81, wherein the mouth leak status is (i) no mouth leak, (ii) valve-like mouth leak, or (iii) continuous mouth leak.

Alternative Implementation 83. The method of Alternative Implementation 82, wherein the no mouth leak is associated with a full face mask, a nasal mask, or a pillows mask.

Alternative Implementation 84. The method of any one of Alternative Implementations 82 to 83, wherein the valve-like mouth leak is associated with a nasal mask or a pillows mask.

Alternative Implementation 85. The method of any one of Alternative Implementations 82 to 84, wherein the continuous mouth leak is associated with a full face mask, a nasal mask, or a pillows mask.

Alternative Implementation 86. The method of any one of Alternative Implementations 55 to 85, wherein the pressurized air supplied to the airway of the user during the therapy session is between 4 cmH2O to 20 cmH2O.

Alternative Implementation 87. The method of Alternative Implementation 86, wherein the pressurized air supplied to the airway of the user during the therapy session is about 8 cmH2O.

Alternative Implementation 88. The method of any one of Alternative Implementations 55 to 87, further comprising: calculating a therapy score or AHI score based at least in part on the determined mouth leak status.

Alternative Implementation 89. The method of Alternative Implementation 88, further comprising: receiving, from a sensor coupled to the respiratory device, sensor data associated with the user during the therapy session, the sensor data being indicative of a number of sleep-disordered breathing events during the therapy session; correlating the mouth leak status with the sensor data to output one or more false positive sleep-disordered breathing events; subtracting the one or more false positive sleep-disordered breathing events from the number of sleep-disordered breathing events to output a modified number of sleep-disordered breathing events; and calculating the therapy score based, at least in part, on the modified number of sleep-disordered breathing events.

Alternative Implementation 90. The method of any one of Alternative Implementations 55 to 89, wherein the mouth leak status includes a duration of mouth leak, a severity of mouth leak, or both; and wherein the method further comprises decreasing a sleep score or therapy score based, at least in part, on the duration of mouth leak, the severity of mouth leak, or both.

Alternative Implementation 91. The method of any one of Alternative Implementations 55 to 90, further comprising: providing control signals to the respiratory device; and responsive to the mouth leak status, adjusting pressure settings of the respiratory device, the pressure settings being associated with the pressurized air supplied to the airway of the user.

Alternative Implementation 92. The method of Alternative Implementation 91, further comprising: analyzing the airflow data associated with the user to determine that the user is exhaling; and responsive to the determination that the user is exhaling, reducing a pressure of the pressurized air to the airway of the user during the exhaling of the user.

Alternative Implementation 93. The method of Alternative Implementation 92, wherein the reducing the pressure of the pressurized air includes increasing an Expiratory Pressure Relief (EPR) level associated with the respiratory device.

Alternative Implementation 94. The method of any one of Alternative Implementations 55 to 93, further comprising: providing control signals to a humidifier coupled to the respiratory device, the humidifier being configured to introduce moisture to the pressurized air supplied to the airway of the user; and responsive to the mouth leak status, adjusting humidification settings associated with the humidifier such that more moisture is introduced into the pressurized air supplied to the airway of the user.

Alternative Implementation 95. The method of Alternative Implementation 94, further comprising releasing a portion of a decongestant into the moisture to be introduced into the pressurized air for the adjusting the humidification settings.

Alternative Implementation 96. The method of any one of Alternative Implementations 55 to 95, further comprising: providing control signals to a smart pillow; and responsive to the mouth leak status, adjusting the smart pillow such that the smart pillow urges the user to change position of the user's head.

Alternative Implementation 97. The method of any one of Alternative Implementations 55 to 96, further comprising: providing control signals to a smart bed or a smart mattress; and responsive to the mouth leak status, adjusting the smart bed or the smart mattress such that the smart bed or the smart mattress urges the user to change position of the user's body.

Alternative Implementation 98. The method of any one of Alternative Implementations 55 to 97, further comprising: providing control signals to a wearable sensor, the wearable sensor being couplable to a body part of the user; and responsive to the mouth leak status, adjusting the wearable sensor such that the wearable sensor stimulates a neck or a jaw of the user to close the user's mouth.

Alternative Implementation 99. The method of any one of Alternative Implementations 55 to 98, further comprising: responsive to the mouth leak status, causing a notification to be provided to the user via an electronic device, such that the user is alerted of the mouth leak status.

Alternative Implementation 100. The method of Alternative Implementation 99, wherein the electronic device is an electronic display device and the providing the notification includes displaying, on the electronic display device, a message.

Alternative Implementation 101. The method of Alternative Implementation 100, wherein the electronic display device is a mobile phone.

Alternative Implementation 102. The method of any one of Alternative Implementations 99 to 101, wherein the notification includes a reminder for the user to (i) close his/her mouth during the therapy session, (ii) moisturize lips before a next therapy session, or (iii) both (i) and (ii).

Alternative Implementation 103. The method of any one of Alternative Implementations 99 to 102, wherein the notification includes an instruction and/or recommendation to the user (i) to use a different mask, (ii) to wake up, (iii) that the user is having a mouth leak, or a combination thereof.

Alternative Implementation 104. The method of any one of Alternative Implementations 99 to 103, wherein the electronic device includes a speaker and the providing the notification includes playing, via the speaker, sound.

Alternative Implementation 105. The method of Alternative Implementation 104, wherein the sound is loud enough to wake up the user.

Alternative Implementation 106. The method of any one of Alternative Implementations 55 to 105, wherein the mouth leak status includes a mouth leak score for the therapy session.

Alternative Implementation 107. The method of Alternative Implementation 106, wherein the mouth leak score is determined based, at least in part, on a percentage of mouth leak during the therapy session, a mouth leak peak volume, a mouth leak total volume, or a combination thereof.

Alternative Implementation 108. The method of Alternative Implementation 106 or Alternative Implementation 107, further comprising: receiving, from a user device, user input data indicative of subjective feedback associated with the user; and determining the mouth leak score based, at least in part, on the user input data.

Alternative Implementation 109. The method of any one of Alternative Implementations 55 to 108, further comprising: receiving sleep stage data associated with the user during the therapy session; determining a sleep stage based at least in part on the sleep stage data; and associate the mouth leak status with the sleep stage.

Alternative Implementation 110. The method of Alternative Implementation 109, wherein the sleep stage includes wake, drowsy, sleep, light sleep, deep sleep, N1 sleep, N2 sleep, N3 sleep, REM sleep, sleep stage fragmentation, or a combination thereof.

Alternative Implementation 111. The method of Alternative Implementation 109 or Alternative Implementation 110, further comprising: causing an indication to be displayed on a display device, the indication including a separate mouth leak status per sleep stage.

Alternative Implementation 112. A system comprising: a control system including one or more processors; and a memory having stored thereon machine readable instructions; wherein the control system is coupled to the memory, and the method of any one of Alternative Implementations 55 to 111 is implemented when the machine executable instructions in the memory are executed by at least one of the one or more processors of the control system.

Alternative Implementation 113. A system for determining a mouth leak status associated with a user of a respiratory device, the system including a control system configured to implement the method of any one of Alternative Implementations 55 to 111.

Alternative Implementation 114. A computer program product comprising instructions which, when executed by a computer, cause the computer to carry out the method of any one of Alternative Implementations 55 to 111.

Alternative Implementation 115. The computer program product of Alternative Implementation 114, wherein the computer program product is a non-transitory computer readable medium.

One or more elements or aspects or steps, or any portion(s) thereof, from one or more of any of claims 1-65 and/or one or more of any of the alternative implementations 1-115 herein can be combined with one or more elements or aspects or steps, or any portion(s) thereof, from one or more of any of the other claims 1-65, one or more of any of the alternative implementations 1-115, or combinations thereof, to form one or more additional implementations and/or claims of the present disclosure.

While the present disclosure has been described with reference to one or more particular embodiments or implementations, those skilled in the art will recognize that many changes may be made thereto without departing from the spirit and scope of the present disclosure. Each of these implementations and obvious variations thereof is contemplated as falling within the spirit and scope of the present disclosure. It is also contemplated that additional implementations according to aspects of the present disclosure may combine any number of features from any of the implementations described herein.

**Claims**

1. A system comprising:

    a control system (110) including one or more processors (112); and
    a memory (114) having stored thereon machine readable instructions;
    wherein the control system (110) is coupled to the memory (114), and a method is implemented when the machine executable instructions in the memory (114) are executed by at least one of the one or more processors (112) of the control system (110), the method comprising:

        receiving airflow data associated with the user (210) of a respiratory device (122), the respiratory device (122) being configured to supply pressurized air to an airway of the user (210) during a therapy session, the airflow data including pressure data and/or flow rate data;
        analyzing the airflow data associated with the user, wherein the analyzing the airflow data associated with the user (210) includes processing the airflow data to identify one or more features that distinguish mouth leak from (i) normal respiration during therapy and/or (ii) other types of unintentional leak, and wherein the one or more features include a covariance between unintentional leak and ventilation; and
        based at least in part on the analysis, determining the mouth leak status associated with the user, the mouth leak status being indicative of whether or not air is leaking from a mouth of the user.

2. The system of claim 1, wherein the one or more features further includes a time the covariance holds above a threshold, a normalized ventilation, an unintentional leak variability, a normalized respiration rate, a respiration rate variability, or any combination thereof.

3. The system of claim 1 or claim 2, wherein the one or more features are computed on a user flow rate signal, a mask pressure signal, a blower flow rate signal, a blower pressure signal, or any combination thereof; wherein the one or more features include, for each signal, (i) a frame area, (ii) a breath area, (iii) a complement to the breath area, (iv) a ratio of the breath area over the frame area, (v) a ratio of the breath area over the complement to the breath area, (vi) a skewness of the signal, (vii) a kurtosis of the signal, (viii) a first derivative of the skewness, (ix) a first derivative of the kurtosis, (x) a second derivative of the skewness, (xi) a second derivative of the kurtosis, or (xii) any combination thereof.

4. The system of any one of claims 1 to 3, wherein:

the one or more features includes a minimum pressure, a maximum pressure, a pressure skewness, a pressure kurtosis, a pressure power spectral density, a flow rate range, a minimum flow rate, a maximum flow rate, a flow skewness, a flow kurtosis, a flow sub-area ratio, or any combination thereof;
optionally, the minimum pressure is associated with an end of the inhalation portion, a beginning of the exhalation portion, or both; and
optionally, the flow sub-area ratio is calculated by dividing a first sub-area from a second sub-area, the first sub-area being a portion of a flow expiratory area, the second sub-area being the flow expiratory area, wherein the flow expiratory area is delimited by a flow expiratory curve and zero flow rate, wherein the portion of the flow expiratory area is delimited by the flow expiratory curve and a flow threshold level.

5. The system of claim 4, wherein the mouth leak status is determined based, at least in part, on the pressure range, a detrended minimum pressure, and the flow sub-area ratio.

6. The system of any one of claims 4 or 5, wherein the mouth leak status is determined based, at least in part, on an output from a logistic regression model, and wherein the logistic regression model can be calculated by:

$$p = \frac{1}{1 + e^{-(b + \alpha_1 x_1 + \alpha_2 x_2 + \alpha_3 x_3)}}$$

and, optionally, wherein the output from the logistic regression model greater than or equal to a threshold is indicative of the mouth leak status being valve-like mouth leak or continuous mouth leak

7. The system of any one of claims 1 to 6, further comprising determining an operational mode of the respiratory device (122), wherein the one or more features are determined based at least in part on the determined operational mode, and, optionally wherein the one or more features are determined based at least in part on removing an Expiratory Pressure Relief (EPR) component in the pressure data.

8. The system of any one of claims 1 to 7, wherein the mouth leak status is (i) no mouth leak, (ii) valve-like mouth leak, or (iii) continuous mouth leak; and/or

the method executed by at least one of the one or more processors (112) of the control system (110) further comprises calculating a therapy score or AHI score based at least in part on the determined mouth leak status, and optionally,

receiving, from a sensor coupled to the respiratory device, sensor data associated with the user during the therapy session, the sensor data being indicative of a number of sleep-disordered breathing events during the therapy session;
correlating the mouth leak status with the sensor data to output one or more false positive sleep-disordered breathing events;
subtracting the one or more false positive sleep-disordered breathing events from the number of sleep-disordered breathing events to output a modified number of sleep-disordered breathing events; and
calculating the therapy score based, at least in part, on the modified number of sleep-disordered breathing events; and/or.

wherein the mouth leak status includes a duration of mouth leak, a severity of mouth leak, or both; and wherein the method further comprises decreasing a sleep score or therapy score based, at least in part, on the duration of mouth leak, the severity of mouth leak, or both.

9. The system of any one of claims 1 to 8, further comprising:

providing control signals to the respiratory device; and
responsive to the mouth leak status, adjusting pressure settings of the respiratory device, the pressure settings being associated with the pressurized air supplied to the airway of the user; and optionally
analyzing the airflow data associated with the user to determine that the user is exhaling; and
responsive to the determination that the user is exhaling, reducing a pressure of the pressurized air to the airway of the user during the exhaling of the user.

10. The system of any one of claims 1 to 9, further comprising:

providing control signals to a humidifier (129) coupled to the respiratory device (122), the humidifier (129) being configured to introduce moisture to the pressurized air supplied to the airway of the user (210); and
responsive to the mouth leak status, adjusting humidification settings associated with the humidifier (129) such that more moisture is introduced into the pressurized air supplied to the airway of the user (210).

11. The system of any one of claims 1 to 10, further comprising:

providing control signals to a smart pillow, a smart bed or a smart mattress, a wearable sensor couplable to a body part of the user (210), or a combination thereof; and
responsive to the mouth leak status, determining an adjustment the smart pillow such that the smart pillow urges the user (210) to change position of the user's head, determining an adjustment to the smart bed or the smart mattress such that the smart bed or the smart mattress urges the user (210) to change position of the user's body, determining an adjustment to the wearable sensor such that the wearable sensor stimulates a neck or a jaw of the user to (210) close the user's mouth, or a combination thereof.

12. The system of any one of claims 1 to 11, further comprising responsive to the mouth leak status, causing a notification to be provided to the user via an electronic device, such that the user (210), physician and/or health care provider is alerted of the mouth leak status.

13. The system of any one of claims 1 to 12, wherein the mouth leak status includes a mouth leak score for the therapy session, and the mouth leak score is determined based, at least in part, on a percentage of mouth leak during the therapy session, a mouth leak peak volume, a mouth leak total volume, or a combination thereof.

14. The system of any one of claims 1 to 13, wherein a signed covariance between unintentional leak (1420) and ventilation (1430) is used to isolate onset and offset of mouth leak events.

15. The system of any one of claims 1 to 14, further comprising a respiratory therapy device (120).

**FIG. 1**

FIG. 2A

FIG. 2B

300

310

Receive acoustic data associated with a user of a respiratory device during a sleep session

320

Analyze the acoustic data associated with the user

330

Determine a mouth leak status of the user

331

Adjust AHI score

332

Adjust pressure settings of the respiratory device

333

Adjust humidification settings associated with a humidifier

334

Adjust a smart pillow to urge the user to change position of their head

335

Adjust a smart mattress to urge the user to change position of their body

336

Stimulate a neck or a jaw of the user to close their mouth

337

Provide a notification and/ or a message to the user

340

Display the mouth leak status of the user on a display device

**FIG. 3**

EP 4 697 353 A2

**Jane's Mouth
Leak Rating**

Wake 😊 😐 ☹
Light Sleep 😊 😐 ☹
Deep Sleep 😊 😐 ☹
REM Sleep 😊 😐 ☹

Continue

**FIG. 4A**

9:41

You are mouth breathing

**Please switch to
full-face mask**

Continue

**FIG. 4B**

9:41

Tired?

(YES) (NO) (MAYBE)

Congestion?

(YES) (NO) (MAYBE)

Dry mouth?

(YES) (NO) (MAYBE)

Add symptom

**FIG. 4C**

500

510

Receive inhalation pressure data and exhalation pressure data associated with pressurized air supplied to a user during a plurality of sleep sessions

520

Receive inhalation acoustic data and exhalation acoustic data associated with the user during the plurality of sleep sessions

530

Analyze the inhalation acoustic data and the exhalation acoustic data associated with the user

540

Determine an optimal inhalation pressure and an optimal exhalation pressure for the user

550

Set the optimal inhalation pressure and the optimal exhalation pressure as the pressure settings for the pressurized air supplied to the user for a subsequent sleep session

FIG. 5

600

610
Receive acoustic data associated with a user of a respiratory device during a plurality of sleep sessions

620
Receive physiological data associated with the user for the plurality of sleep sessions

630
Analyze the acoustic data to determine a mouth leak status of the user for each sleep session of the plurality of sleep sessions

640
Train a machine learning algorithm

650
Input current physiological data during a current sleep session into the MLA

MLA

652
Output estimated mouth leak status for the current sleep session

654
Adjust pressure settings of the respiratory device

660
Input current physiological data prior to a next sleep session into the MLA

MLA

662
Output estimated mouth leak status for the next sleep session

664
Determine a recommended adjustment for the user

FIG. 6

700

710

Receive airflow data associated with
the user of the respiratory device.

720

Analyze the airflow data associated with the user.

722

Identify at least a first breath
cycle of the user

730

Determine the
mouth leak status
associated with
the user.

724

Process the airflow data to
identify one or more features
associated with the at least
first breath cycle

740

Determine an operational
mode of the respiratory
device

FIG. 7

**FIG. 8**

**FIG. 9**

EP 4 697 353 A2

**FIG. 10A**

Legend:
- tidal_volume (L/s)
- leak (L/s)
- patient_flow (L/s)
- mask_pressure/3-2 (cmH20)

VML

Mask Leak

CML

1010

1020

1030

Time (s)

Legend:
- tidal_volume (L/s)
- leak (L/s)
- patient_flow (L/s)
- mask_pressure/3-2 (cmH20)

1010

**FIG. 10B**

EP 4 697 353 A2

**FIG. 10C**

**FIG. 10D**

Legend:
- tidal_volume (L/s)
- leak (L/s)
- patient_flow (L/s)
- mask_pressure/3-2 (cmH20)

1030

FIG. 11

FIG. 12A

FIG. 12B

FIG. 13

1410 Mouth leak flag

1420 Un-intentional leak [L/s]

1430 3 min ventilation [L]

1440 Signed covariance

Time [h]

FIG. 14

**Feature separation for ventilation on levels of unintentional leak**

FIG. 15

FIG. 16A

FIG. 16B

FIG. 17

Patient with ML: high levels of unintentional leak, small leak variance

FIG. 18A

Patient with no ML: leak variance is high for high levels of mask leak

FIG. 18B

FIG. 19

FIG. 20A

Other features:
Areas between the line min-max and patient flow (their ratio)

FIG. 20B

FIG. 21

FIG. 22

FIG. 23

No mask leak        Mask leak

FIG. 24A

No mask leak        Mask leak

FIG. 24B

FIG. 25

Continuous      Continuous
mouth leak      mouth leak          *Mask leak*

FIG. 26A

Continuous          Continuous
mouth leak          mouth leak              *Mask leak*

FIG. 26B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62968889
- US 63198137
- WO 2016061629 A
- WO 2018050913 A
- WO 2020104465 A
- WO 2012012835 A
- WO 2015006364 A

**Non-patent literature cited in the description**

- **CHILDERS et al.** *Proceedings of the IEEE*, October 1977, vol. 65 (10)
- **RANDALL RB**. *Frequency Analysis, Copenhagen: Bruel & Kjaer*, 1977, 344